# EUROPÄISCHE PATENTSCHRIFT

(11) **EP 3 341 349 B1**
(45) Veröffentlichungstag und Bekanntmachung des Hinweises auf die Patenterteilung: **29.01.2020**
(21) Anmeldenummer: 16753582.2
(22) Anmeldetag: 29.07.2016
(51) Int. Cl.: C07D 307/91, C07C 13/62, C09K 11/06, H01L 51/50

(54) **6,9,15,18-TETRAHYDRO-S-INDACENO[1,2-B:5,6-B']DIFLUOREN- DERIVATE UND IHRE VERWENDUNG IN ELEKTRONISCHEN VORRICHTUNGEN**
6,9,15,18-TETRAHYDRO-S-INDACENO[1,2-B:5,6-B']DIFLUORENE- DERIVATIVES AND THEIR USE IN ELECTRONIC DEVICES
DÉRIVÉS DE 6,9,15,18-TÉTRAHYDRO-S-INDACÉNO[1,2-B:5,6-B']DIFLUORÈNE ET LEUR UTILISATION DANS ET DES DISPOSITIFS ÉLECTRONIQUES

(30) Priorität: 28.08.2015 EP 15182993
(43) Veröffentlichungstag der Anmeldung: 04.07.2018
(73) Patentinhaber: Merck Patent GmbH, 64293 Darmstadt (DE)
(72) Erfinder: HEIL, Holger, 60316 Frankfurt am Main (DE); BURKHART, Beate, 64293 Darmstadt (DE); RODRIGUEZ, Lara-Isabel, 64285 Darmstadt (DE); MEYER, Sebastian, 63741 Aschaffenburg (DE); LINGE, Rouven, 64291 Darmstadt (DE); DARSY, Amandine, 60320 Frankfurt am Main (DE)
(86) Internationale Anmeldenummer: PCT/EP2016/001320
(87) Internationale Veröffentlichungsnummer: WO 2017/036574

(56) Entgegenhaltungen:
- WO-A1-2006/122630
- WO-A1-2010/012328
- WO-A2-2014/111269

## Beschreibung

Die vorliegende Erfindung betrifft eine Verbindung einer Formel (II-1), bzw. Formel (II-2), sowie Ihre Verwendung in elektronischen Vorrichtungen, insbesondere in organischen elektronischen Vorrichtungen (OLEDs). Weiterhin betrifft die Erfindung bestimmte Ausführungsformen von elektronischen Vorrichtungen enthaltend die Verbindung der Formel (II-1), bzw. Formel (II-2), sowie Verfahren zur Herstellung der Verbindung der Formel (II-1), bzw. Formel (II-2).

Unter dem Begriff elektronische Vorrichtung werden gemäß der vorliegenden Erfindung allgemein elektronische Vorrichtungen verstanden, welche organische Materialien enthalten. Bevorzugt werden darunter OLEDs verstanden.

Der allgemeine Aufbau sowie das Funktionsprinzip von OLEDs ist dem Fachmann bekannt und unter anderem in US 4539507, US 5151629, EP 0676461 und WO 1998/27136 beschrieben.

Hinsichtlich der Leistungsdaten der elektronischen Vorrichtungen sind weitere Verbesserungen erforderlich, insbesondere um eine breite kommerzielle Verwendung der elektronischen Vorrichtungen zu ermöglichen, wie beispielsweise in Displays oder als Lichtquellen. In diesem Zusammenhang sind die Lebensdauer, die Effizienz und die Betriebsspannung der elektronischen Vorrichtungen sowie die realisierten Farbwerte von besonderer Bedeutung.

Bei der Nutzung von herkömmlichen weißen Lichtquellen und Monitoren ist derzeit die insgesamt nutzbare Lebensdauer der blau emittierenden Komponente begrenzend. Deshalb besteht bei blau emittierenden OLEDs ein Verbesserungspotential bezüglich der Lebensdauer der elektronischen Vorrichtungen und den erreichten Farbwerten des emittierten Lichts.

Ein wichtiger Ansatzpunkt, um die genannten Verbesserungen zu erreichen, ist die Auswahl der Emitterverbindung, die in der elektronischen Vorrichtung eingesetzt wird.

Im Stand der Technik sind als blau fluoreszierende Emitterverbindungen eine Vielzahl von Verbindungen beschrieben, insbesondere Arylamine mit Indenofluoren-Grundgerüst. Beispiele dafür sind Benzoindenofluorenamine, z.B. gemäß WO 2008/006449 und WO 2007/140847, Indenofluorenamine bzw. -diamine, beispielsweise gemäß WO 2006/122630, sowie die in WO 2010/012328 offenbarten Indenofluorenderivate mit kondensierten Arylgruppen.

Weiterhin ist in WO 2007/018007 die Verwendung von Stickstoffenthaltenen heterocyclischen Derivaten beschrieben. Die beschriebenen Verbindungen werden als Emitter in einer OLED verwendet, die eine blaue Emission aufweist. Um eine blaue Emission zu erzeugen benötigt die in der WO 2007/018007 genannte elektronische Vorrichtung jedoch eine hohe Betriebsspannung von 6.0 V.

Darüber hinaus ist in EP 1860097 eine Vielzahl von aromatischen Aminderivaten beschrieben, darunter insbesondere Verbindungen mit einem Indenofluoren-Grundgerüst. Die Indenofluoren-Verbindungen werden in der Lochtransportschicht in einer OLED verwendet, die eine blaue Emission aufweist. Um eine blaue Emission zu erzeugen benötigt die in EP 1860097 genannte elektronische Vorrichtung jedoch eine hohe Betriebsspannung von 6.5 V.

In der WO 2014/111269 sind Verbindungen mit einem Benzindenofluoren-Grundgerüst beschrieben. Die beschriebenen Verbindungen werden als Emitter in der emittierenden Schicht einer elektronischen Vorrichtung, wie z.B. OLED, verwendet, welche eine blaue Emission aufweist. Um eine Verwendung der elektronischen Vorrichtung in Displays oder Lichtquellen zu gewährleisten ist es vorteilhaft, die Effizienz der genannten elektronischen Vorrichtung zu steigern.

Zusammenfassend liegt also die technische Aufgabe vor, blau fluoreszierende Emitter bereitzustellen. Insbesondere besteht Bedarf an Verbindungen, mit denen vorteilhafte Leistungsdaten für die elektronischen Vorrichtungen erreicht werden können. Weiterhin bevorzugt liegt die Aufgabe vor, Verbindungen bereitzustellen, mit denen eine niedrige Betriebsspannung, eine hohe Leistungseffizienz, eine lange Lebensdauer und/oder eine blaue Emission der elektronischen Vorrichtung erreicht werden kann, in welche die Verbindungen eingebracht werden.

In Untersuchungen zu neuen Verbindungen zur Verwendung in elektronischen Vorrichtungen wurde nun unerwartet gefunden, dass Verbindungen einer Formel (II-1), bzw. Formel (II-2), mit einem erweiterten Bis-Indenofluoren-Grundgerüst hervorragend zur Verwendung in elektronischen Vorrichtungen geeignet sind und insbesondere blaue Farbkoordinaten aufweisen und dadurch die oben vorgestellte technische Aufgabe lösen. Blaue Farbkoordinaten bei Emitterverbindungen sind hoch erwünscht für die Verwendung in Displays und Beleuchtungsanwendungen und sind ferner hoch erwünscht für die Abstimmung der Farbeindrücke der verschiedenen Farben in einem Display oder bei einer Beleuchtungsanwendung .

Gegenstand der Erfindung ist somit eine Verbindung gemäß einer Formel (II-1) oder Formel (II-2), 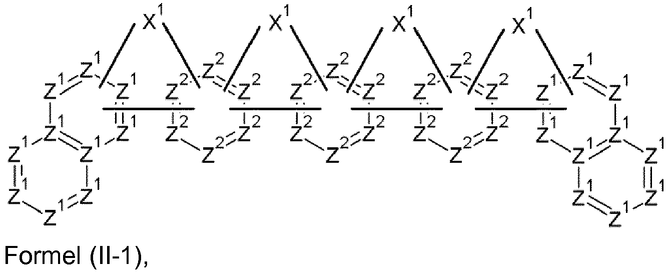 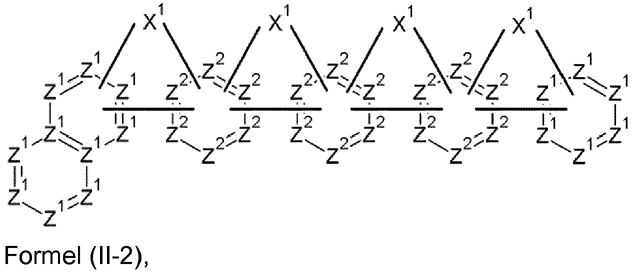 wobei gilt:
- Z¹: ist bei jedem Auftreten gleich oder verschieden CR¹ oder N, wobei Z¹ gleich C ist, wenn eine Gruppe gebunden ist;
- Z²: ist bei jedem Auftreten gleich oder verschieden CR² oder N, wobei Z² gleich C ist, wenn eine Gruppe gebunden ist; und
- X¹: ist bei jedem Auftreten gleich oder verschieden BR³, C(R³)₂, C(R³)₂-C(R³)₂-, C(R³)₂-O-, C(R³)₂-S-, -R³C=CR³⁻, R³C=N-, Si(R³)₂, Ge(R³)2, -Si(R³)₂-Si(R₃)₂-, C=O, O, S, Se, S=O, SO₂, NR³, PR³ oder P(=O)R³, wobei zwei oder mehr Reste R³ miteinander verknüpft sein können und einen Ring bilden können;
- R¹: ist bei jedem Auftreten gleich oder verschieden H, D, F, CN, Si(R⁴)₃, eine geradkettige Alkylgruppe mit 1 bis 20 C-Atomen, eine verzweigte oder cyclische Alkylgruppe mit 3 bis 20 C-Atomen, oder ein aromatisches oder heteroaromatisches Ringsystem mit 5 bis 20 aromatischen Ringatomen, wobei die oben genannten Gruppen jeweils mit einem oder mehreren Resten R⁴ substituiert sein können, und wobei eine oder mehrere CH₂-Gruppen in den oben genannten Gruppen durch -C≡C-, -R⁴C=CR⁴, Si(R⁴)₂, C=O, R⁴, O oder S ersetzt sein kann;
- R², R³: ist bei jedem Auftreten gleich oder verschieden H, D, F, Cl, Br, I, C(=O)R⁴, CN, Si(R⁴)₃, N(R⁴)₂, P(=O)(R⁴)₂, OR⁴, S(=O)R⁴, S(=O)₂R⁴, eine geradkettige Alkyl- oder Alkoxygruppe mit 1 bis 20 C-Atomen, eine verzweigte oder cyclische Alkyl- oder Alkoxygruppe mit 3 bis 20 C-Atomen, eine Alkenyl- oder Alkinylgruppe mit 2 bis 20 C-Atomen, ein aromatisches oder heteroaromatisches Ringsystem mit 5 bis 30 aromatischen Ringatomen oder eine Aryloxy- oder Heteroaryloxygruppe mit 5 bis 30 aromatischen Ringatomen, wobei
die oben genannten Gruppen jeweils mit einem oder mehreren Resten R⁴ substituiert sein können, wobei eine oder mehrere CH₂-Gruppen in den oben genannten Gruppen durch -R⁴C=CR⁴-, C≡C, Si(R⁴)2, C=O, C=NR⁴, -C(=O)O-, -C(=O)NR⁴-, R⁴, P(=O)(R⁴), O, S, SO oder SO₂ ersetzt sein können, wobei
ein oder mehrere H-Atome in den oben genannten Gruppen durch D, F, Cl, Br, I oder CN ersetzt sein können;
- R⁴: ist bei jedem Auftreten gleich oder verschieden H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, eine geradkettige Alkyl- oder Alkoxygruppe mit 1 bis 20 C-Atomen, eine verzweigte oder cyclische Alkyl- oder Alkoxygruppe mit 3 bis 20 C-Atomen, eine Alkenyl- oder Alkinylgruppe mit 2 bis 20 C-Atomen, ein aromatisches oder heteroaromatisches Ringsystem mit 5 bis 30 aromatischen Ringatomen oder eine Aryloxy- oder Heteroaryloxygruppe mit 5 bis 30 aromatischen Ringatomen, wobei
die oben genannten Gruppen jeweils mit einem oder mehreren Resten R⁵ substituiert sein können, wobei
eine oder mehrere CH₂-Gruppen in den oben genannten Gruppen durch -R⁵C=CR⁵-, -C≡C-, Si(R⁵)₂, C=O, C=NR⁵, -C(=O)O-, C(=O)NR⁵, NR⁵, P(=O)(R⁵), O, S, SO oder SO₂ ersetzt sein können, wobei
ein oder mehrere H-Atome in den oben genannten Gruppen durch D, F, Cl, Br, I oder CN ersetzt sein können, und wobei
zwei oder mehr Reste R⁴ miteinander verknüpft sein können und einen Ring bilden können;
- R⁵: ist bei jedem Auftreten gleich oder verschieden H, D, F, Cl, Br, I, CN, eine geradkettige Alkyl- oder Alkoxygruppe mit 1 bis 20 C-Atomen, eine verzweigte oder cyclische Alkyl- oder Alkoxygruppe mit 3 bis 20 C-Atomen, eine Alkenyl- oder Alkinylgruppe mit 2 bis 20 C-Atomen, ein aromatisches oder heteroaromatisches Ringsystem mit 5 bis 30 aromatischen Ringatomen, oder eine Aryloxy- oder Heteroaryloxygruppe mit 5 bis 30 aromatischen Ringatomen, wobei
zwei oder mehr Reste R⁵ miteinander verknüpft sein können und einen Ring bilden können;
Es folgen allgemeine Definitionen für chemische Gruppen im Rahmen der vorliegenden Anmeldung:
Eine Arylgruppe im Sinne dieser Erfindung enthält 6 bis 60 aromatische Ringatome. Eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus Stickstoff (N), Sauerstoff (O), Schwefel (S), Silicium (Si) und/oder Phosphor (P), und sind besonders bevorzugt ausgewählt aus Stickstoff (N), Sauerstoff (O) und/oder Schwefel (S). Dies stellt die grundlegende Definition dar. Werden in der Beschreibung der vorliegenden Erfindung andere Bevorzugungen angegeben, beispielsweise bezüglich der Zahl der aromatischen Ringatome oder der Zahl oder Art der enthaltenen Heteroatome, so gelten diese.

Dabei wird unter einer Arylgruppe bzw. einer Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Gruppen substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol, oder eine Kombination dieser Gruppen.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 5 bis 60 aromatische C-Atome im Ringsystem. Ein heteroaromatisches Ringsystem im Sinne dieser Erfindung enthält 5 bis 60 aromatische Ringatome, von denen mindestens ein Ringatom ein Heteroatom darstellt. Die Heteroatome sind bevorzugt ausgewählt aus Stickstoff (N), Sauerstoff (O), Schwefel (S), Silicium (Si) und/oder Phosphor (P), und sind besonders bevorzugt ausgewählt aus Stickstoff (N), Sauerstoff (O) und/oder Schwefel (S). Dies stellt die grundlegende Definition dar. Werden in der Beschreibung der vorliegenden Erfindung andere Bevorzugungen angegeben, beispielsweise bezüglich der Zahl der aromatischen Ringatome oder der Zahl oder Art der enthaltenen Heteroatome, so gelten diese. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nichtaromatische Einheit (bevorzugt weniger als 10 % der von H verschiedenen Atome), wie z. B. ein sp³-hybridisiertes C-, Si-, N- oder O-Atom, ein sp²⁻hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Biphenyl, Terphenyl oder Diphenyltriazin.

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol, oder eine Kombination dieser Gruppen.

Im Rahmen der vorliegenden Erfindung werden unter einer geradkettigen Alkylgruppe mit 1 bis 20 C-Atomen, bzw. einer verzweigten oder cyclischen Alkylgruppe mit 3 bis 20 C-Atomen, bzw. einer Alkenyl- oder Alkinylgruppe mit 2 bis 20 C-Atomen, eine Gruppe bezeichnet in der auch einzelne H-Atome oder CH₂-Gruppen durch die oben bei der Definition der Reste genannten Gruppen substituiert sein können. Dies stellt die grundlegende Definition dar. Werden in der Beschreibung der vorliegenden Erfindung andere Bevorzugungen angegeben, beispielsweise bezüglich der Zahl der C-Atome, so gelten diese. Bevorzugt werden unter einer geradkettigen Alkylgruppe mit 1 bis 20 C-Atomen, bzw. einer verzweigten oder cyclischen Alkylgruppe mit 3 bis 20 C-Atomen, bzw. einer Alkenyl- oder Alkinylgruppe mit 2 bis 20 C-Atomen, die Gruppen Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 20 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht: 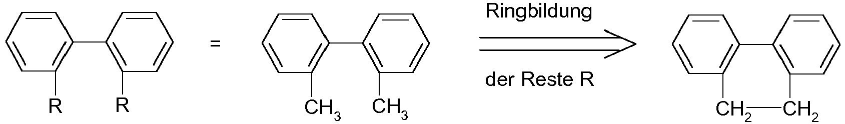 Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden: 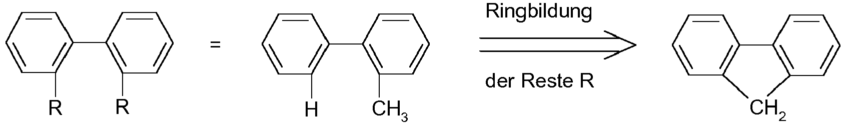 Unter der Formulierung, dass zwei oder mehr Reste miteinander einen Ring bilden können, soll im Rahmen der vorliegenden Anmeldung unter anderem verstanden werden, dass die beiden Reste eine Arylgruppe, eine Hetroarylgruppe, eine Aryloxygruppe, eine Heteroaryloxygruppe, ein aromatisches oder heteroaromatisches Ringsystem, eine cyclische Alkyl-, Alkenyl-, oder Alkinylgruppe im Sinne dieser Erfindung bilden können.

Es ist bevorzugt, dass X¹ bei jedem Auftreten gleich oder verschieden gewählt ist aus C(R³)₂, -C(R³)₂-C(R³)₂-, -C(R³)₂-O-, -R³C=CR³-, Si(R³)₂, C=O, O, S, S=O, SO₂, und NR³, wobei zwei oder mehr Reste R³ miteinander verknüpft sein können und einen Ring bilden können. Besonders bevorzugt ist X¹ ausgewählt aus C(R³)₂, -C(R³)₂-C(R³)₂-, -C(R³)₂-O-, Si(R³)₂, O, S, und NR³, wobei zwei oder mehr Reste R³ miteinander verknüpft sein können und einen Ring bilden können. Ganz besonders bevorzugt ist X¹ gleich C(R³)₂.

Bevorzugt ist R² bei jedem Auftreten gleich oder verschieden H, D, F, CN, Si(R⁴)₃, eine geradkettige Alkyl- oder Alkoxygruppe mit 1 bis 20 C-Atomen, eine verzweigte oder cyclische Alkyl- oder Alkoxygruppe mit 3 bis 20 C-Atomen, oder ein aromatisches oder heteroaromatisches Ringsystem mit 5 bis 20 aromatischen Ringatomen, wobei die oben genannten Gruppen jeweils mit einem oder mehreren Resten R⁴ substituiert sein können, und wobei eine oder mehrere CH₂-Gruppen in den oben genannten Gruppen durch -C≡C-, -R⁴C=CR⁴, Si(R⁴)2, C=O, R⁴, O oder S ersetzt sein kann.

Besonders bevorzugt ist R¹ bei jedem Auftreten gleich oder verschieden gewählt aus H, CN und einem aromatischen oder heteroaromatischen Ringsystem mit 5 bis 30 aromatischen Ringatomen, wobei die oben genannten Gruppen jeweils mit einem oder mehreren Resten R⁴ substituiert sein können.

Besonders bevorzugt ist R² gleich H oder D, besonders bevorzugt gleich H.

Bevorzugt ist R³ bei jedem Auftreten gleich oder verschieden F, CN, Si(R⁴)₃, eine geradkettige Alkyl- oder Alkoxygruppe mit 1 bis 20 C-Atomen, eine verzweigte oder cyclische Alkyl- oder Alkoxygruppe mit 3 bis 20 C-Atomen, oder ein aromatisches oder heteroaromatisches Ringsystem mit 5 bis 20 aromatischen Ringatomen, wobei die oben genannten Gruppen jeweils mit einem oder mehreren Resten R⁴ substituiert sein können, wobei eine oder mehrere CH₂-Gruppen in den oben genannten Gruppen durch -C≡C-, -R⁴C=CR⁴-, Si(R⁴)₂, C=O, R⁴, O oder S ersetzt sein können.

Gemäß einer bevorzugten Ausführungsform ist R³ bei jedem Auftreten gleich oder verschieden eine geradkettige Alkylgruppe mit 1 bis 20 C-Atomen oder eine verzweigte Alkylgruppe mit 3 bis 20 C-Atomen. Besonders bevorzugt ist R³ eine geradkettige Alkylgruppe mit 5 bis 12 C-Atomen.

Gemäß einer bevorzugten Ausführungsform bilden zwei Reste R³, die Bestandteil einer Gruppe X¹ sind, die C(R³)₂ oder Si(R³)₂ darstellt, miteinander einen Ring, so dass eine Spiro-Verbindung entsteht. Bevorzugt wird dabei ein Fünf- oder ein Sechsring gebildet. Weiterhin ist es in diesem Fall bevorzugt, dass die Reste R³ Alkylgruppen darstellen, so dass ein spirocyclischer Alkylring gebildet wird, besonders bevorzugt ein Spiro-Cyclohexanring oder ein Spiro-Cyclopentanring.

Bevorzugt ist R⁴ bei jedem Auftreten gleich oder verschieden F, CN, Si(R⁵)₃, eine geradkettige Alkyl- oder Alkoxygruppe mit 1 bis 20 C-Atomen, eine verzweigte oder cyclische Alkyl- oder Alkoxygruppe mit 3 bis 20 C-Atomen, oder ein aromatisches oder heteroaromatisches Ringsystem mit 5 bis 20 aromatischen Ringatomen, wobei die oben genannten Gruppen jeweils mit einem oder mehreren Resten R⁵ substituiert sein können, wobei eine oder mehrere CH₂-Gruppen in den oben genannten Gruppen durch -C≡C-, -R⁵C=CR⁵-, Si(R⁵)₂, C=O, NR⁵, O und S ersetzt sein können oder wobei zwei oder mehr Reste R⁴ miteinander verknüpft sein können und einen Ring bilden können.

Gemäß einer bevorzugten Ausführungsform der Erfindung sind alle Gruppen R¹ und R² in Formel (II-1), bzw. Formel (II-2) gleich H oder D, besonders bevorzugt gleich H.

Gemäß einer weiteren bevorzugten Ausführungsform der Erfindung sind eine oder mehrere Gruppen R¹ gleich CN, besonders bevorzugt genau zwei Gruppen R¹ gleich CN.

Gemäß einer weiteren bevorzugten Ausführungsform der Erfindung sind eine oder mehrere Gruppen R¹ gleich einem aromatischen oder heteroaromatischen Ringsystem mit 6 bis 20 aromatischen Ringatomen, das mit einem oder mehreren Resten R⁴ substituiert sein kann, besonders bevorzugt genau zwei Gruppen R¹ gleich einem aromatischen oder heteroaromatischen Ringsystem mit 6 bis 20 aromatischen Ringatomen, das mit einem oder mehreren Resten R⁴ substituiert sein kann.

Gemäß einer besonders bevorzugten Ausführungsform ist R¹ gleich Phenyl, Naphthyl, Carbazol, Benzocarbazol, Dibenzofuran, Benzofuran, Fluoren oder Anthracen, welches jeweils mit Resten R⁴ substituiert sein kann.

Gemäß einer weiteren bevorzugten Ausführungsform der Erfindung sind keiner der Gruppen R² Gruppen der Formel N(R⁴)₂. In diesem Fall sind bevorzugt die Gruppen X¹ nicht gleich NR³, besonders bevorzugt sind die Gruppen X¹ in diesem Fall gleich C(R³)₂.

Für die Formel (II-1) und (II-2) gilt, dass die Bindungen zu Gruppen X¹ und die Bindungen zwischen den aromatischen Ringen jeweils an beliebigen Positionen der aromatischen Ringe vorliegen können, ebenso wie die Bindungen zwischen den einzelnen aromatischen Ringen. Insbesondere wird durch die Darstellung der Formeln (II-1) und (II-2) nicht impliziert, dass die Gruppen X¹ in cis-Stellung zueinander vorliegen müssen. Die Gruppen X¹ können in cis- oder in trans-Stellung zueinander vorliegen.

Es ist bevorzugt, dass maximal zwei Gruppen Z¹ pro aromatischem Ring gleich N sind, besonders bevorzugt maximal eine Gruppe Z¹ pro aromatischem Ring gleich N ist, und ganz besonders bevorzugt keine Gruppe Z¹ in einem aromatischen Ring gleich N ist.

Allgemein bevorzugt ist, dass Z¹ gleich CR¹ ist.

Es ist bevorzugt, dass maximal zwei Gruppen Z² pro aromatischem Ring gleich N sind, besonders bevorzugt maximal eine Gruppe Z² pro aromatischem Ring gleich N ist, und ganz besonders bevorzugt keine Gruppe Z² in einem aromatischen Ring gleich N ist.

Allgemein bevorzugt ist, dass Z² gleich CR² ist.

Bevorzugte Ausführungsformen der Formeln (II-1), bzw. (II-2) entsprechen den folgenden Formeln (II-1-1) bis (II-1-20), bzw. (II-2-1) bis (II-2-9)

| |
|---|
| 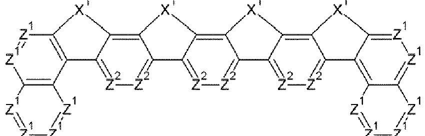 |
| Formel (II-1-1) |
| 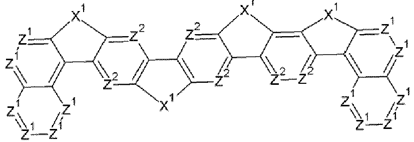 |
| Formel (II-1-2) |
| 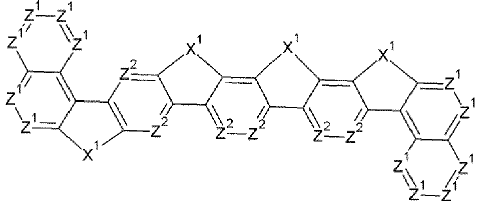 |
| Formel (II-1-3) |
| 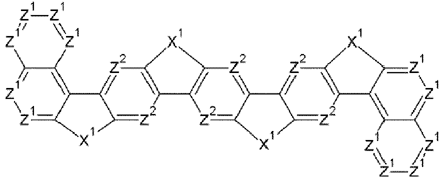 |
| Formel (II-1-4) |
| 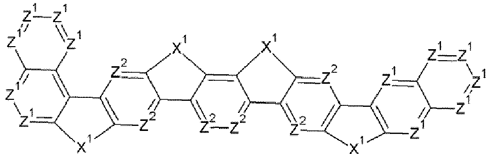 |
| Formel (II-1-5) |
| 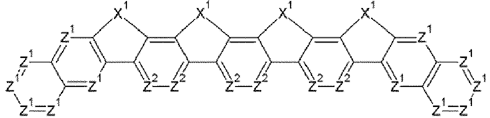 |
| Formel (II-1-6) |
| 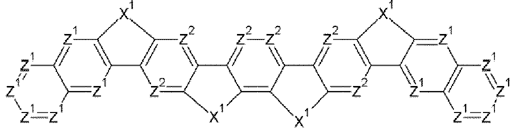 |
| Formel (II-1-7) |
| 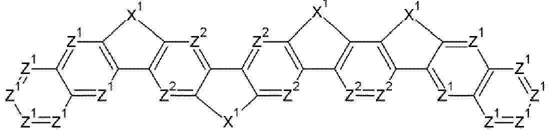 |
| Formel (II-1-8) |
| 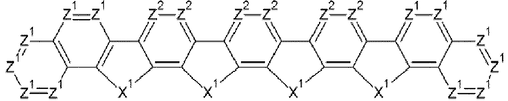 |
| Formel (II-1-9) |
| 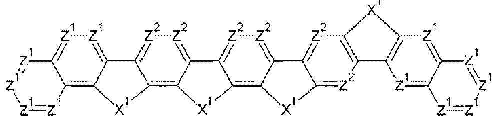 |
| Formel (II-1-10) |
| 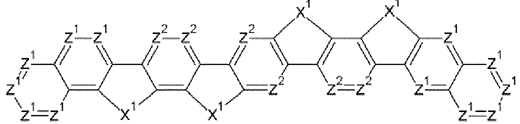 |
| Formel (II-1-11) |
| 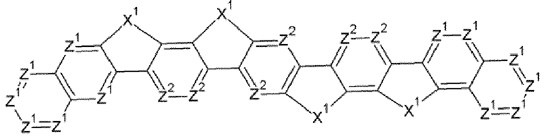 |
| Formel (II-1-12) |
| 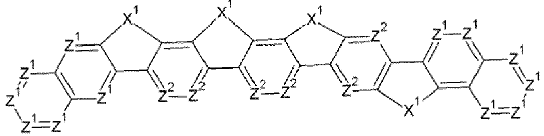 |
| Formel (II-1-13) |
| 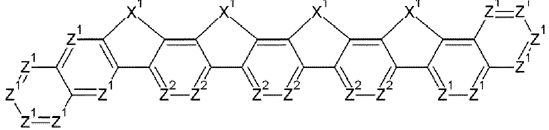 |
| Formel (II-1-14) |
| 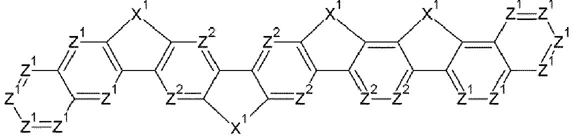 |
| Formel (II-1-15) |
| 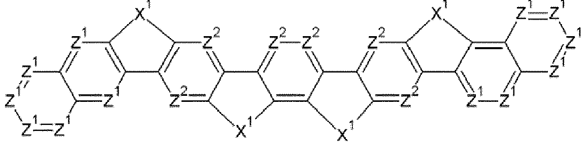 |
| Formel (II-1-16) |
| 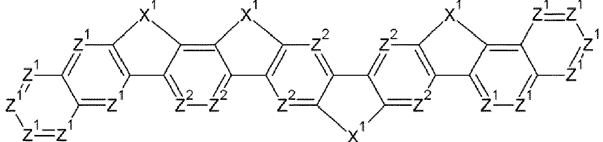 |
| Formel (II-1-17) |
| 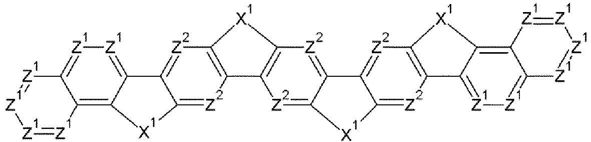 |
| Formel (II-1-18) |
| 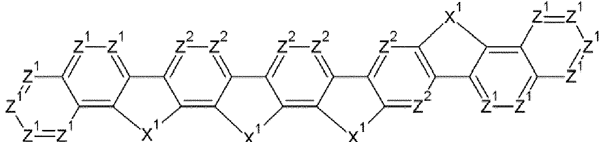 |
| Formel (II-1-19) |
| 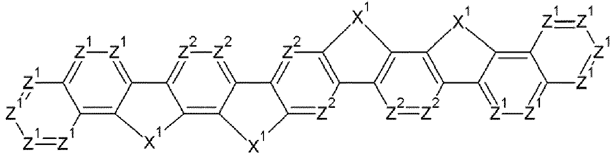 |
| Formel (II-1-20) |
| 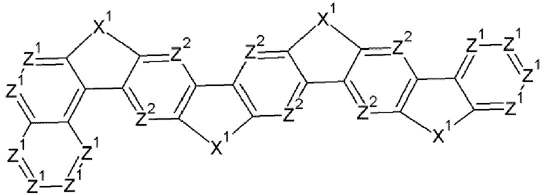 |
| Formel (II-2-1) |
| 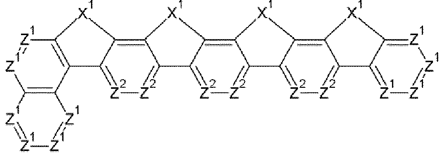 |
| Formel (II-2-2) |
| 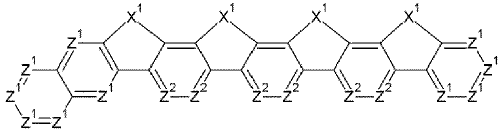 |
| Formel (II-2-3) |
| 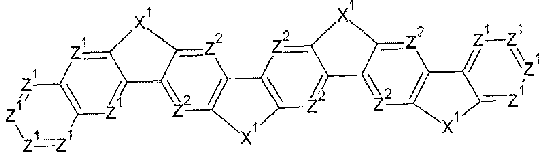 |
| Formel (II-2-4) |
| 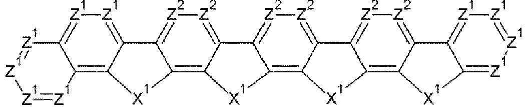 |
| Formel (II-2-5) |
| 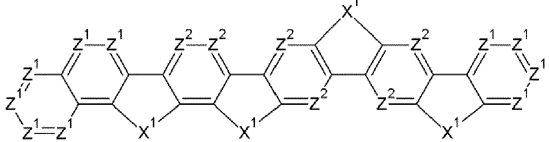 |
| Formel (II-2-6) |
| 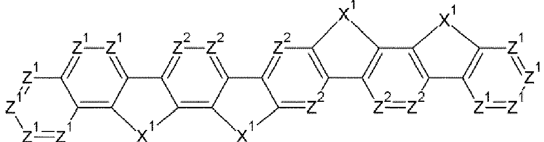 |
| Formel (II-2-7) |
| 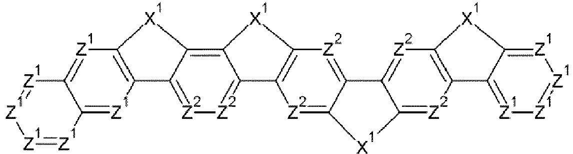 |
| Formel (II-2-8) |
| 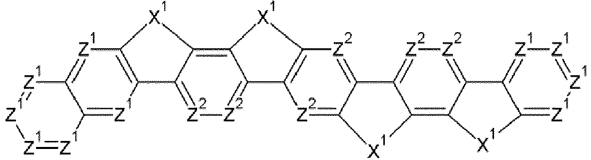 |
| Formel (II-2-9) |

wobei gilt:
- Z¹: ist bei jedem Auftreten gleich oder verschieden CR¹ oder N;
- Z²: ist bei jedem Auftreten gleich oder verschieden CR² oder N; und
die Gruppen X¹ sind definiert wie oben.

Insbesondere für die Gruppen Z¹, Z² und X¹ sind die oben angegebenen bevorzugten Ausführungsformen auch für die obenstehenden Formeln bevorzugt.

Nochmals insbesondere bevorzugt ist in den Formeln (II-1-1) bis (II-1-20) und (II-2-1) bis (II-2-9) Z¹ gleich CR¹, Z² ist gleich CR², und X¹ ist gleich C(R³)₂.

Unter den Formeln (II-1-1) bis (11-1-20) und (II-2-1) bis (II-2-9) ist die Formel (11-1-4) besonders bevorzugt.

Bevorzugt entsprechen Verbindungen der Formel (II) der Formel (II-1-4-1).

| |
|---|
| 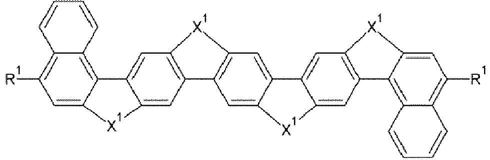 |
| Formel (II-1-4-1) |

wobei X¹ und R¹ wie oben definiert sind.

Bevorzugt ist X¹ in der Formel (11-1-4-1) bei jedem Auftreten gleich oder verschieden gewählt aus C(R³)₂, -C(R³)₂-C(R³)₂-, -C(R³)₂-O-, -Si(R³)₂, O, S, und NR³, besonders bevorzugt ist X¹ gleich C(R³)₂.

Bevorzugt ist R¹ in der Formel (11-1-4-1) bei jedem Auftreten gleich oder verschieden H, D, F, CN, Si(R⁴)₃ oder ein aromatisches oder heteroaromatisches Ringsystem mit 5 bis 20 aromatischen Ringatomen, das jeweils mit einem oder mehreren Resten R⁴ substituiert sein kann.

Gemäß einer besonders bevorzugten Ausführungsform ist R¹ in der Formel (II-1-4-1) gleich Phenyl, Naphthyl, Carbazol, Benzocarbazol, Dibenzofuran, Benzofuran, Fluoren oder Anthracen, welches jeweils mit Resten R⁴ substituiert sein kann.

Ganz besonders bevorzugte Ausführungsformen der Verbindungen der Formel (II), entsprechen den folgenden Formeln, wobei bevorzugt gilt: Z¹ ist CR¹ und Z² ist CR²_{:}

| | Grundstruktur gemäß | X¹ (links) | X¹ (Mitte, links) | X¹ (Mitte, rechts) | X¹ (rechts) |
|---|---|---|---|---|---|
| 1 | Formel (II-1-1) | C(R³)₂ | C(R³)₂ | C(R³)₂ | C(R³)₂ |
| 2 | " | O | C(R³)₂ | C(R³)₂ | O |
| 3 | " | N(R³)₂ | C(R³)₂ | C(R³)₂ | N(R³)₂ |
| 4 | " | C(R³)₂ | N(R³)₂ | N(R³)₂ | C(R³)₂ |
| 5 | " | C(R³)₂ | O | N(R³)₂ | C(R³)₂ |
| 6 | Formel (II-1-2) | C(R³)₂ | C(R³)₂ | C(R³)₂ | C(R³)₂ |
| 7 | " | O | C(R³)₂ | C(R³)₂ | O |
| 8 | " | N(R³)₂ | C(R³)₂ | C(R³)₂ | N(R³)₂ |
| 9 | " | C(R³)₂ | N(R³)₂ | N(R³)₂ | C(R³)₂ |
| 10 | " | C(R³)₂ | O | N(R³)₂ | C(R³)₂ |
| 11 | Formel (II-1-3) | C(R³)₂ | C(R³)₂ | C(R³)₂ | C(R³)₂ |
| 12 | " | O | C(R³)₂ | C(R³)₂ | O |
| 13 | " | N(R³)₂ | C(R³)₂ | C(R³)₂ | N(R³)₂ |
| 14 | " | C(R³)₂ | N(R³)₂ | N(R³)₂ | C(R³)₂ |
| 15 | " | C(R³)₂ | O | N(R³)₂ | C(R³)₂ |
| 16 | Formel (II-1-4) | C(R³)₂ | C(R³)₂ | C(R³)₂ | C(R³)₂ |
| 17 | " | O | C(R³)₂ | C(R³)₂ | O |
| 18 | " | N(R³)₂ | C(R³)₂ | C(R³)₂ | N(R³)₂ |
| 19 | " | C(R³)₂ | N(R³)₂ | N(R³)₂ | C(R³)₂ |
| 20 | " | C(R³)₂ | O | N(R³)₂ | C(R³)₂ |
| 21 | Formel (II-1-5) | C(R³)₂ | C(R³)₂ | C(R³)₂ | C(R³)₂ |
| 22 | " | O | C(R³)₂ | C(R³)₂ | O |
| 23 | " | N(R³)₂ | C(R³)₂ | C(R³)₂ | N(R³)₂ |
| 24 | " | C(R³)₂ | N(R³)₂ | N(R³)₂ | C(R³)₂ |
| 35 | " | C(R³)₂ | O | N(R³)₂ | C(R³)₂ |
| 26 | Formel (II-1-6) | C(R³)₂ | C(R³)₂ | C(R³)₂ | C(R³)₂ |
| 27 | " | O | C(R³)₂ | C(R³)₂ | O |
| 28 | " | N(R³)₂ | C(R³)₂ | C(R³)₂ | N(R³)₂ |
| 29 | " | C(R³)₂ | N(R³)₂ | N(R³)₂ | C(R³)₂ |
| 30 | " | C(R³)₂ | O | N(R³)₂ | C(R³)₂ |
| 31 | Formel (II-1-7) | C(R³)₂ | C(R³)₂ | C(R³)₂ | C(R³)₂ |
| 32 | " | O | C(R³)₂ | C(R³)₂ | O |
| 33 | " | N(R³)₂ | C(R³)₂ | C(R³)₂ | N(R³)₂ |
| 34 | " | C(R³)₂ | N(R³)₂ | N(R³)₂ | C(R³)₂ |
| 35 | " | C(R³)₂ | O | N(R³)₂ | C(R³)₂ |
| 36 | Formel (II-1-8) | C(R³)₂ | C(R³)₂ | C(R³)₂ | C(R³)₂ |
| 37 | " | O | C(R³)₂ | C(R³)₂ | O |
| 38 | " | N(R³)₂ | C(R³)₂ | C(R³)₂ | N(R³)₂ |
| 39 | " | C(R³)₂ | N(R³)₂ | N(R³)₂ | C(R³)₂ |
| 40 | " | C(R³)₂ | O | N(R³)₂ | C(R³)₂ |
| 41 | Formel (II-1-9) | C(R³)₂ | C(R³)₂ | C(R³)₂ | C(R³)₂ |
| 42 | " | O | C(R³)₂ | C(R³)₂ | O |
| 43 | " | N(R³)₂ | C(R³)₂ | C(R³)₂ | N(R³)₂ |
| 44 | " | C(R³)₂ | N(R³)₂ | N(R³)₂ | C(R³)₂ |
| 45 | " | C(R³)₂ | O | N(R³)₂ | C(R³)₂ |
| 46 | Formel (II-1-10) | C(R³)₂ | C(R³)₂ | C(R³)₂ | C(R³)₂ |
| 47 | " | O | C(R³)₂ | C(R³)₂ | O |
| 48 | " | N(R³)₂ | C(R³)₂ | C(R³)₂ | N(R³)₂ |
| 49 | " | C(R³)₂ | N(R³)₂ | N(R³)₂ | C(R³)₂ |
| 50 | " | C(R³)₂ | O | N(R³)₂ | C(R³)₂ |
| 51 | Formel (II-1-11) | C(R³)₂ | C(R³)₂ | C(R³)₂ | C(R³)₂ |
| 52 | " | O | C(R³)₂ | C(R³)₂ | O |
| 53 | " | N(R³)₂ | C(R³)₂ | C(R³)₂ | N(R³)₂ |
| 54 | " | C(R³)₂ | N(R³)₂ | N(R³)₂ | C(R³)₂ |
| 55 | " | C(R³)₂ | O | N(R³)₂ | C(R³)₂ |
| 56 | Formel (II-1-12) | C(R³)₂ | C(R³)₂ | C(R³)₂ | C(R³)₂ |
| 57 | " | O | C(R³)₂ | C(R³)₂ | O |
| 58 | " | N(R³)₂ | C(R³)₂ | C(R³)₂ | N(R³)₂ |
| 59 | " | C(R³)₂ | N(R³)₂ | N(R³)₂ | C(R³)₂ |
| 60 | " | C(R³)₂ | O | N(R³)₂ | C(R³)₂ |
| 61 | Formel (II-1-13) | C(R³)₂ | C(R³)₂ | C(R³)₂ | C(R³)₂ |
| 62 | " | O | C(R³)₂ | C(R³)₂ | O |
| 63 | " | N(R³)₂ | C(R³)₂ | C(R³)₂ | N(R³)₂ |
| 64 | " | C(R³)₂ | N(R³)₂ | N(R³)₂ | C(R³)₂ |
| 65 | " | C(R³)₂ | O | N(R³)₂ | C(R³)₂ |
| 66 | Formel (II-1-14) | C(R³)₂ | C(R³)₂ | C(R³)₂ | C(R³)₂ |
| 67 | " | O | C(R³)₂ | C(R³)₂ | O |
| 68 | " | N(R³)₂ | C(R³)₂ | C(R³)₂ | N(R³)₂ |
| 69 | " | C(R³)₂ | N(R³)₂ | N(R³)₂ | C(R³)₂ |
| 70 | " | C(R³)₂ | O | N(R³)₂ | C(R³)₂ |
| 71 | Formel (II-1-15) | C(R³)₂ | C(R³)₂ | C(R³)₂ | C(R³)₂ |
| 72 | " | O | C(R³)₂ | C(R³)₂ | O |
| 73 | " | N(R³)₂ | C(R³)₂ | C(R³)₂ | N(R³)₂ |
| 74 | " | C(R³)₂ | N(R³)₂ | N(R³)₂ | C(R³)₂ |
| 75 | " | C(R³)₂ | O | N(R³)₂ | C(R³)₂ |
| 76 | Formel (II-1-16) | C(R³)₂ | C(R³)₂ | C(R³)₂ | C(R³)₂ |
| 77 | " | O | C(R³)₂ | C(R³)₂ | O |
| 78 | " | N(R³)₂ | C(R³)₂ | C(R³)₂ | N(R³)₂ |
| 79 | " | C(R³)₂ | N(R³)₂ | N(R³)₂ | C(R³)₂ |
| 80 | " | C(R³)₂ | O | N(R³)₂ | C(R³)₂ |
| 81 | Formel (II-1-17) | C(R³)₂ | C(R³)₂ | C(R³)₂ | C(R³)₂ |
| 82 | " | O | C(R³)₂ | C(R³)₂ | O |
| 83 | " | N(R³)₂ | C(R³)₂ | C(R³)₂ | N(R³)₂ |
| 84 | " | C(R³)₂ | N(R³)₂ | N(R³)₂ | C(R³)₂ |
| 85 | " | C(R³)₂ | O | N(R³)₂ | C(R³)₂ |
| 86 | Formel (II-1-18) | C(R³)₂ | C(R³)₂ | C(R³)₂ | C(R³)₂ |
| 87 | " | O | C(R³)₂ | C(R³)₂ | O |
| 88 | " | N(R³)₂ | C(R³)₂ | C(R³)₂ | N(R³)₂ |
| 89 | " | C(R³)₂ | N(R³)₂ | N(R³)₂ | C(R³)₂ |
| 90 | " | C(R³)₂ | O | N(R³)₂ | C(R³)₂ |
| 91 | Formel (II-1-19) | C(R³)₂ | C(R³)₂ | C(R³)₂ | C(R³)₂ |
| 92 | " | O | C(R³)₂ | C(R³)₂ | O |
| 93 | " | N(R³)₂ | C(R³)₂ | C(R³)₂ | N(R³)₂ |
| 94 | " | C(R³)₂ | N(R³)₂ | N(R³)₂ | C(R³)₂ |
| 95 | " | C(R³)₂ | O | N(R³)₂ | C(R³)₂ |
| 96 | Formel (II-1-20) | C(R³)₂ | C(R³)₂ | C(R³)₂ | C(R³)₂ |
| 97 | " | O | C(R³)₂ | C(R³)₂ | O |
| 98 | " | N(R³)₂ | C(R³)₂ | C(R³)₂ | N(R³)₂ |
| 99 | " | C(R³)₂ | N(R³)₂ | N(R³)₂ | C(R³)₂ |
| 100 | " | C(R³)₂ | O | N(R³)₂ | C(R³)₂ |
| 101 | Formel (II-2-1) | C(R³)₂ | C(R³)₂ | C(R³)₂ | C(R³)₂ |
| 102 | " | O | C(R³)₂ | C(R³)₂ | O |
| 103 | " | N(R³)₂ | C(R³)₂ | C(R³)₂ | N(R³)₂ |
| 104 | " | C(R³)₂ | N(R³)₂ | N(R³)₂ | C(R³)₂ |
| 105 | " | C(R³)₂ | O | N(R³)₂ | C(R³)₂ |
| 106 | Formel (II-2-2) | C(R³)₂ | C(R³)₂ | C(R³)₂ | C(R³)₂ |
| 107 | " | O | C(R³)₂ | C(R³)₂ | O |
| 108 | " | N(R³)₂ | C(R³)₂ | C(R³)₂ | N(R³)₂ |
| 109 | " | C(R³)₂ | N(R³)₂ | N(R³)₂ | C(R³)₂ |
| 110 | " | C(R³)₂ | O | N(R³)₂ | C(R³)₂ |
| 111 | Formel (II-2-3) | C(R³)₂ | C(R³)₂ | C(R³)₂ | C(R³)₂ |
| 112 | " | O | C(R³)₂ | C(R³)₂ | O |
| 113 | " | N(R³)₂ | C(R³)₂ | C(R³)₂ | N(R³)₂ |
| 114 | " | C(R³)₂ | N(R³)₂ | N(R³)₂ | C(R³)₂ |
| 115 | " | C(R³)₂ | O | N(R³)₂ | C(R³)₂ |
| 116 | Formel (II-2-4) | C(R³)₂ | C(R³)₂ | C(R³)₂ | C(R³)₂ |
| 117 | " | O | C(R³)₂ | C(R³)₂ | O |
| 118 | " | N(R³)₂ | C(R³)₂ | C(R³)₂ | N(R³)₂ |
| 119 | " | C(R³)₂ | N(R³)₂ | N(R³)₂ | C(R³)₂ |
| 120 | " | C(R³)₂ | O | N(R³)₂ | C(R³)₂ |
| 121 | Formel (II-2-5) | C(R³)₂ | C(R³)₂ | C(R³)₂ | C(R³)₂ |
| 122 | " | O | C(R³)₂ | C(R³)₂ | O |
| 123 | " | N(R³)₂ | C(R³)₂ | C(R³)₂ | N(R³)₂ |
| 124 | " | C(R³)₂ | N(R³)₂ | N(R³)₂ | C(R³)₂ |
| 125 | " | C(R³)₂ | O | N(R³)₂ | C(R³)₂ |
| 126 | Formel (II-2-6) | C(R³)₂ | C(R³)₂ | C(R³)₂ | C(R³)₂ |
| 127 | " | O | C(R³)₂ | C(R³)₂ | O |
| 128 | " | N(R³)₂ | C(R³)₂ | C(R³)₂ | N(R³)₂ |
| 129 | " | C(R³)₂ | N(R³)₂ | N(R³)₂ | C(R³)₂ |
| 130 | " | C(R³)₂ | O | N(R³)₂ | C(R³)₂ |
| 131 | Formel (II-2-7) | C(R³)₂ | C(R³)₂ | C(R³)₂ | C(R³)₂ |
| 132 | " | O | C(R³)₂ | C(R³)₂ | O |
| 133 | " | N(R³)₂ | C(R³)₂ | C(R³)₂ | N(R³)₂ |
| 134 | " | C(R³)₂ | N(R³)₂ | N(R³)₂ | C(R³)₂ |
| 135 | " | C(R³)₂ | O | N(R³)₂ | C(R³)₂ |
| 136 | Formel (II-2-8) | C(R³)₂ | C(R³)₂ | C(R³)₂ | C(R³)₂ |
| 137 | " | O | C(R³)₂ | C(R³)₂ | O |
| 138 | " | N(R³)₂ | C(R³)₂ | C(R³)₂ | N(R³)₂ |
| 139 | " | C(R³)₂ | N(R³)₂ | N(R³)₂ | C(R³)₂ |
| 140 | " | C(R³)₂ | O | N(R³)₂ | C(R³)₂ |
| 141 | Formel (II-2-9) | C(R³)₂ | C(R³)₂ | C(R³)₂ | C(R³)₂ |
| 142 | " | O | C(R³)₂ | C(R³)₂ | O |
| 143 | " | N(R³)₂ | C(R³)₂ | C(R³)₂ | N(R³)₂ |
| 144 | " | C(R³)₂ | N(R³)₂ | N(R³)₂ | C(R³)₂ |
| 145 | " | C(R³)₂ | O | N(R³)₂ | C(R³)₂ |

Bevorzugt sind die folgenden Verbindungen nach der Formel (II-1), bzw. Formel (II-2):

| | |
|---|---|
| 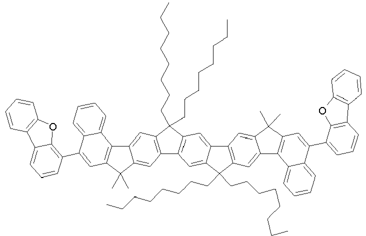 | 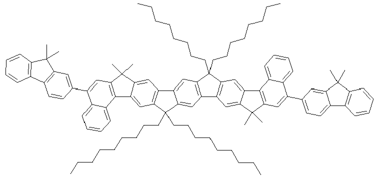 |
| (D1) | (D2) |
| 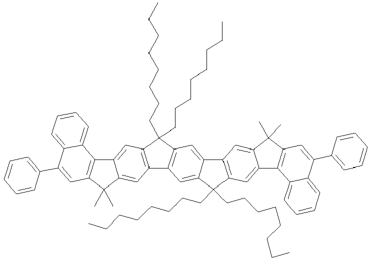 | 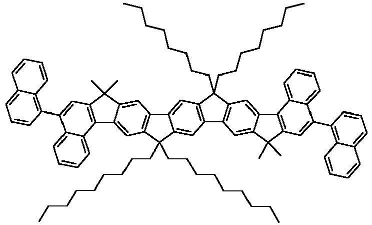 |
| (D3) | (D4) |

Folgende Verbindungen sind Beispiele für Verbindungen der Formel (II-1), bzw. Formel (II-2):

| | |
|---|---|
| 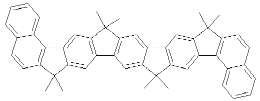 | 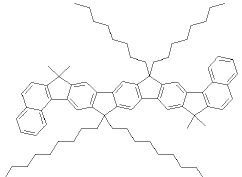 |
| 1 | 2 |
| 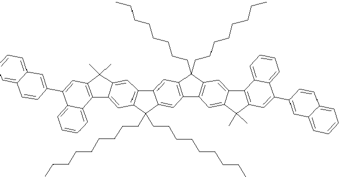 | 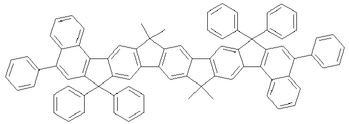 |
| 3 | 4 |
| 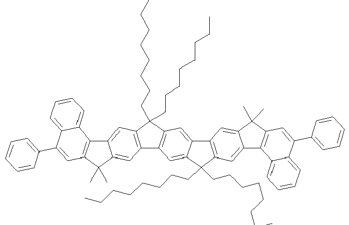 | 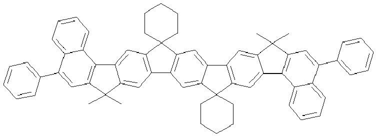 |
| 5 | 6 |
| 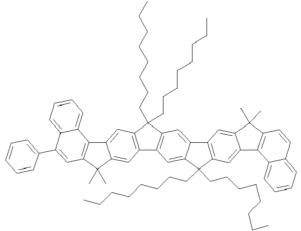 | 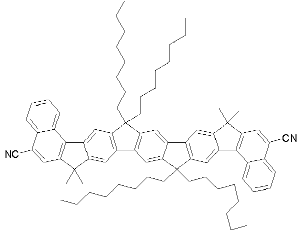 |
| 7 | 8 |
| 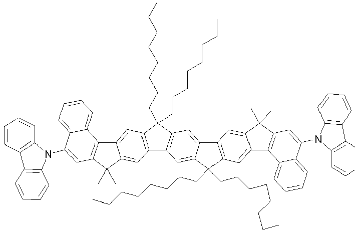 | 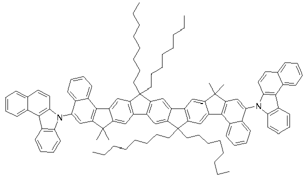 |
| 9 | 10 |
| 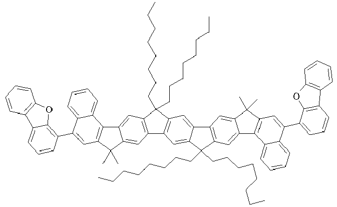 | 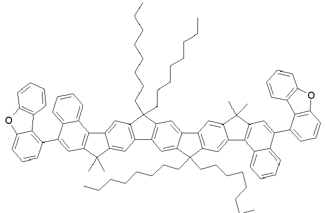 |
| 11 | 12 |
| 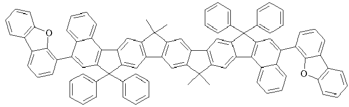 | 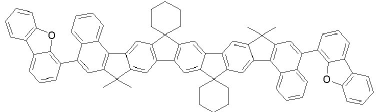 |
| 13 | 14 |
| 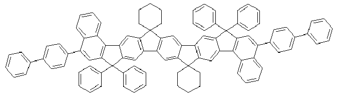 | 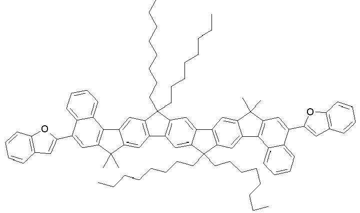 |
| 15 | 16 |
| 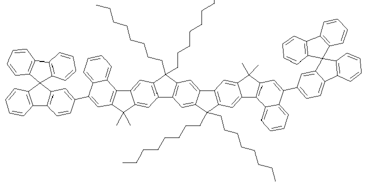 | 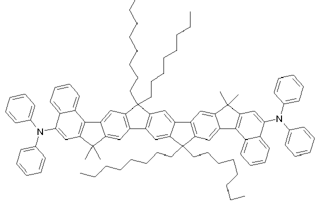 |
| 17 | 18* |
| 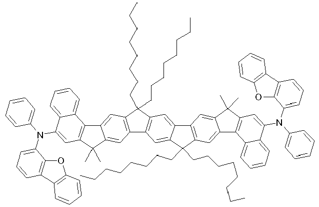 | 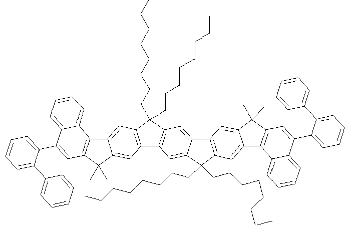 |
| 19* | 20 |
| 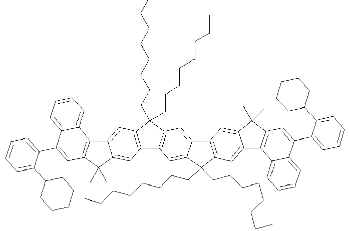 | 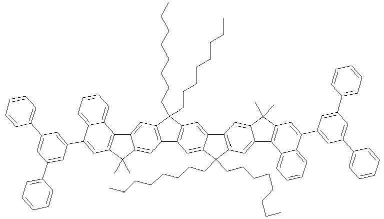 |
| 21 | 22 |
| 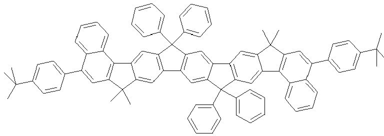 | 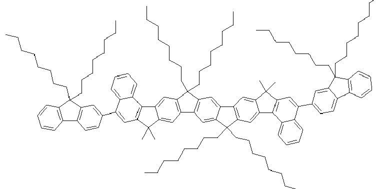 |
| 23 | 24 |
| 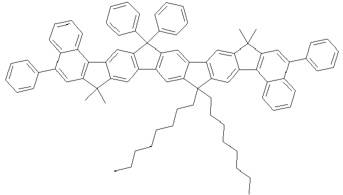 | 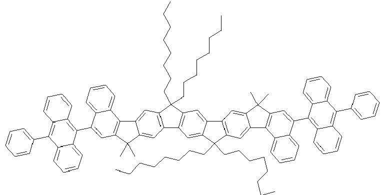 |
| 25 | 26 |
| 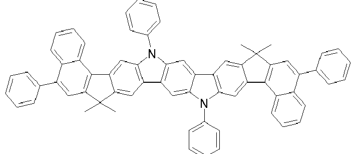 | 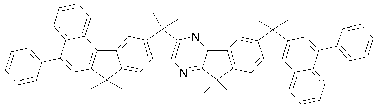 |
| 27 | 28 |
| 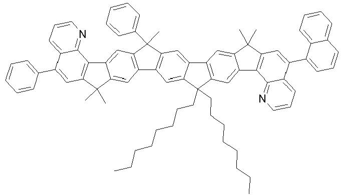 | 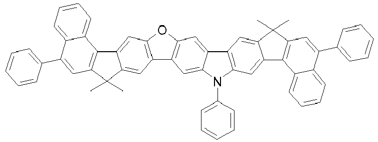 |
| 29 | 30 |
| 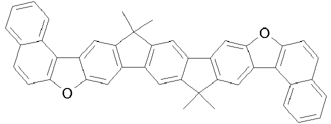 | 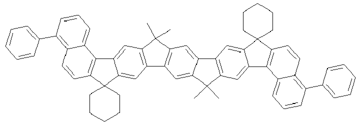 |
| 31 | 32 |
| 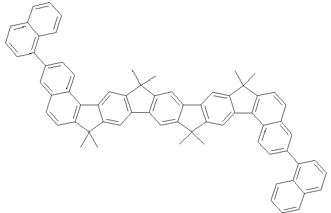 | 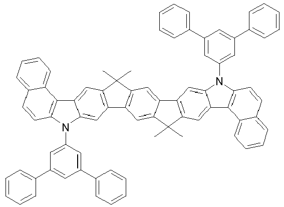 |
| 33 | 34 |
| 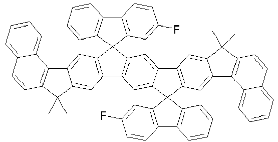 | 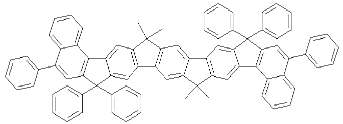 |
| 35 | 36 |
| 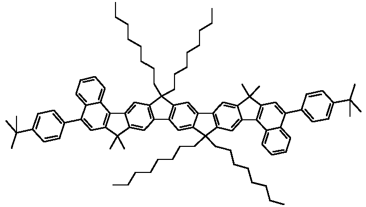 | 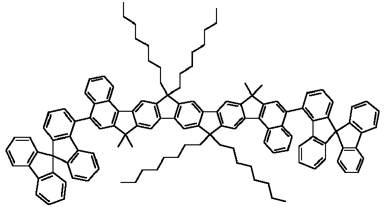 |
| 37 | 38 |
| 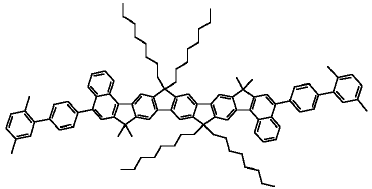 | 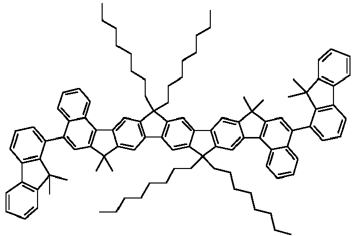 |
| 39 | 40 |
| 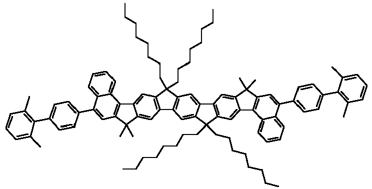 | 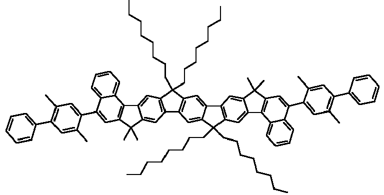 |
| 41 | 42 |
| 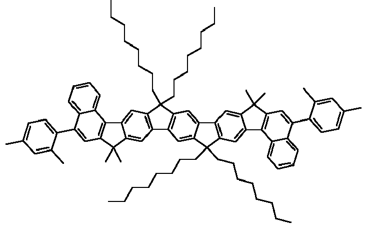 | 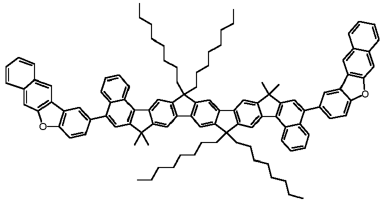 |
| 43 | 44 |
| 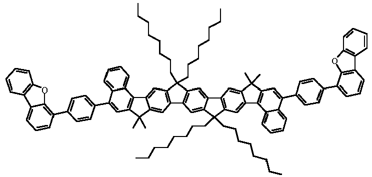 | 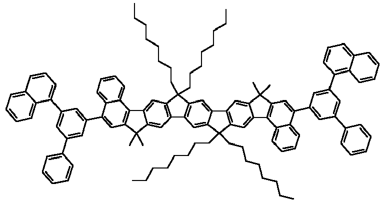 |
| 45 | 46 |
| 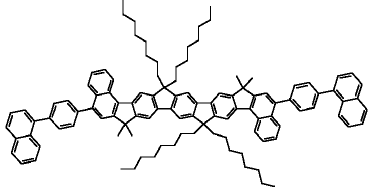 | 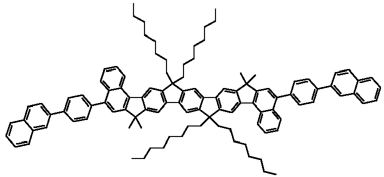 |
| 47 | 48 |
| 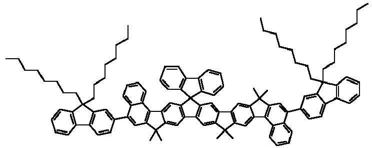 | 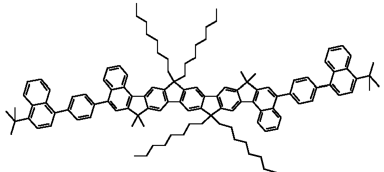 |
| 49 | 50 |
| 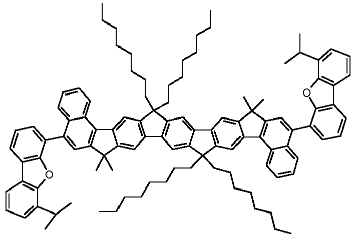 | 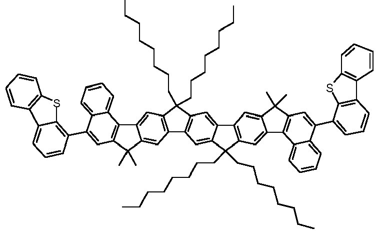 |
| 51 | 52 |
| 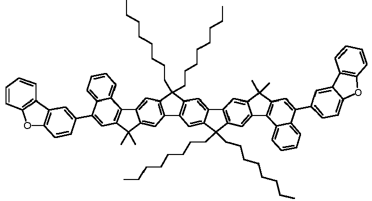 | 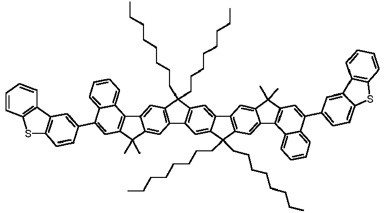 |
| 53 | 54 |
| 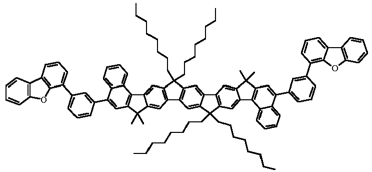 | |
| 55 | |

| | |
|---|---|
| **Nicht erfindungsgemäß* | |

Die Verbindungen der Formel (II-1), bzw. Formel (II-2) können gemäß bekannten Verfahren bzw. Reaktionsschritten der organischen Chemie hergestellt werden.

Ein bevorzugtes Verfahren zur Herstellung von Verbindungen der Formel (II-1), bzw. Formel (II-2) ist im Folgenden gezeigt (Schema 1): 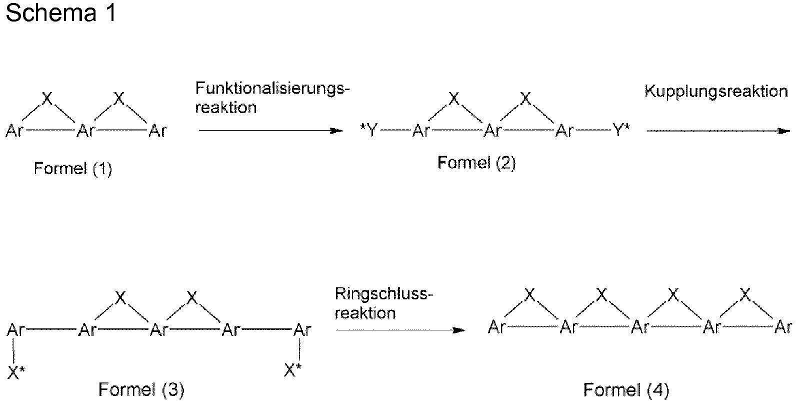
- Ar:: aromatische oder heteroaromatische Gruppe
- X:: verbrückende Gruppe
- X*:: Vorläufergruppe der verbrückenden Gruppe
- Y*:: reaktive Gruppe, beispielsweise Cl, Br, I

Hierzu werden in eine Ausgangsverbindung (Formel 1), die in vielen Fällen kommerziell erhältlich ist, reaktive Gruppen eingeführt, beispielsweise durch Bromierung, oder durch Bromierung und
anschließende Boronierung. Anschließend wird eine doppelte Kupplungsreaktion, beispielsweise eine Suzuki-Kupplungsreaktion, durchgeführt, mit der zwei weitere aromatische Gruppen eingeführt werden. Diese weiteren aromatischen Gruppen enthalten eine funktionelle Gruppe X*, die einen Ringschluss unter Ausbildung einer verbrückenden Gruppe X durchführen kann. Nach der Ringschlussreaktion wird eine Verbindung der Formel (II-1), bzw. Formel (II-2) (Formel 4 in Schema 1), erhalten, welche optional weiter funktionalisiert werden kann.

Details zu den oben schematisch angegebenen Verfahren können den Ausführungsbeispielen entnommen werden.

Der Fachmann kann von den oben schematisch angegebenen Verfahren abweichen oder diese modifizieren, um zu Verbindungen der Formel (II-1), bzw. Formel (II-2) zu gelangen, sofern dies erforderlich ist. Dies erfolgt im Rahmen der üblichen Fähigkeiten des Fachmanns.

Gegenstand der vorliegenden Anmeldung ist somit ein Verfahren zur Herstellung einer Verbindung der Formel (II-1), bzw. Formel (II-2), dadurch gekennzeichnet, dass es mindestens eine metallkatalysierte Kupplungsreaktion und mindestens eine Ringschlussreaktion umfasst. Die metallkatalysierte Kupplungsreaktion ist dabei bevorzugt eine übergangsmetallkatalysierte Kupplungsreaktion, besonders bevorzugt eine Suzuki-Reaktion.

In Schema 2 ist ein Verfahren zur Herstellung einer Verbindung der Formel (II-1), bzw. Formel (II-2) dargestellt.

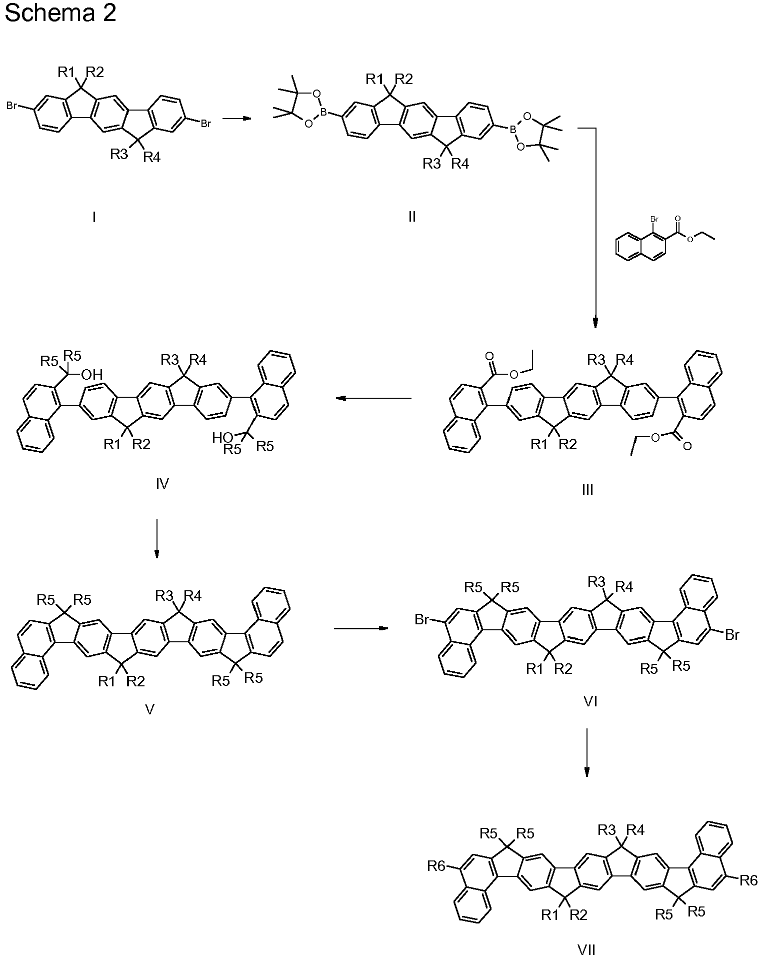

An das oben genannte Syntheseverfahren nach Schema 2 können sich weitere Funktionalisierungsreaktionen anschließen, in denen die erhaltenen erfindungsgemäßen Verbindungen weiter umgesetzt werden.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester substituiert sind, können als Monomere zur Erzeugung entsprechender Oligomere, Dendrimere oder Polymere Verwendung finden. Geeignete reaktive Abgangsgruppen sind beispielsweise Sulfonsäureester, wie z.B. Tosylat oder Triflat, Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Die Erfindung umfasst bevorzugt ein Oligomer, Polymer oder Dendrimer enthaltend eine oder mehrere Verbindungen gemäß Formel (II-1), bzw. Formel (II-2), wobei die Bindung(en) zum Polymer, Oligomer oder Dendrimer an beliebigen, in Formel (II-1), bzw. Formel (II-2) mit R¹ oder R² substituierten Positionen lokalisiert sein können.

Je nach Verknüpfung der Verbindung gemäß Formel (II-1), bzw. Formel (II-2) ist die Verbindung Bestandteil einer Seitenkette oder einer Hauptkette des Oligomers oder Polymers. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten der Formel (II-1), bzw. Formel (II-2) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten der Formel (II-1), bzw. Formel (II-2) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten der Formel (II-1), bzw. Formel (II-2) in Oligomeren, Dendrimeren und Polymeren gelten dieselben Bevorzugungen wie oben für Verbindungen der Formel (II-1), bzw. Formel (II-2) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind ausgewählt aus der Gruppe bestehend aus Fluorenen (z.B. EP 842208 oder WO 2000/22026), Spirobifluorenen (z.B. EP 707020, EP 894107 oder WO 2006/061181), Paraphenylenen (z.B. WO 1992/18552), Carbazolen (z.B. WO 2004/070772 oder WO 2004/113468), Thiophenen (z.B. EP 1028136), Dihydrophenanthrenen (z.B. WO 2005/014689 oder WO 2007/006383), cis- und trans-Indenofluorenen (z.B. WO 2004/041901 oder WO 2004/113412), Ketonen (z.B. WO 2005/040302), Phenanthrenen (z.B. WO 2005/104264 oder WO 2007/017066) oder auch aus mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z.B. WO 2007/068325) oder phosphoreszierende Metallkomplexe (z.B. WO 2006/003000), und/oder Ladungstransporteinheiten, insbesondere solche basierend auf Triarylaminen.

Die erfindungsgemäßen Polymere, Oligomere und Dendrimere werden in der Regel durch Polymerisation von einer oder mehreren Monomersorten hergestellt, von denen mindestens ein Monomer im Polymer zu Wiederholungseinheiten der Formel (II-1), bzw. Formel (II-2) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation;
(D) HARTWIG-BUCHWALD-Polymerisation;
(E) NEGISHI-Polymerisation; und
(F) HIYAMA-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösungsmitteln zu verwenden. Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Gegenstand der Erfindung ist daher weiterhin eine Formulierung, insbesondere eine Lösung, Dispersion oder Emulsion, enthaltend mindestens eine Verbindung der Formel (II-1), bzw. Formel (II-2) oder mindestens ein Polymer, Oligomer oder Dendrimer enthaltend mindestens eine Einheit der Formel (II-1), bzw. Formel (II-2) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und in der darin zitierten Literatur beschrieben.

Die Verbindungen der Formel (II-1), bzw. Formel (II-2) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Die Verbindungen der Formel (II-1), bzw. Formel (II-2) kann in jeder Funktion in der organischen Elektrolumineszenzvorrichtung eingesetzt werden, beispielsweise als lochtransportierendes Material, als Matrixmaterial, als emittierendes Material, oder als elektronentransportierendes Material. Bevorzugt können die Verbindungen der Formel (II-1), bzw. Formel (II-2) als Matrixmaterial oder als emittierendes Material eingesetzt werden, besonders bevorzugt als emittierendes Material.
Weiterer Gegenstand der Erfindung ist daher die Verwendung einer Verbindung gemäß Formel (II-1), bzw. Formel (II-2) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt aus organischen Elektrolumineszenzvorrichtungen (OLEDs).

Weiterer Gegenstand der Erfindung ist eine elektronische Vorrichtung enthaltend mindestens eine Verbindung der Formel (II-1), bzw. Formel (II-2). Bevorzugt ist die elektronische Vorrichtung ausgewählt aus den oben angegebenen Vorrichtungen. Besonders bevorzugt ist eine organische Elektrolumineszenzvorrichtung, enthaltend Anode, Kathode und mindestens eine emittierende Schicht, dadurch gekennzeichnet, dass mindestens eine organische Schicht der organischen Elektrolumineszenzvorrichtung mindestens eine Verbindung gemäß Formel (II-1), bzw. Formel (II-2) oder mindestens ein Oligomer, Polymer oder Dendrimer wie beschrieben enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Dabei muss nicht notwendigerweise jede dieser Schichten vorhanden sein und die Wahl der Schichten hängt von den verwendeten Verbindungen ab und insbesondere auch von der Tatsache ab, ob es sich um eine fluoreszierende oder phosphoreszierende Elektrolumineszenzvorrichtung handelt.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode.
Dabei müssen nicht alle der genannten Schichten vorhanden sein, und es können zusätzlich weitere Schichten vorhanden sein, beispielsweise eine Elektronenblockierschicht anodenseitig an die emittierende Schicht angrenzend, oder eine Lochblockierschicht kathodenseitig an die emittierende Schicht angrenzend.

Bevorzugt umfasst der Gegenstand der Erfindung eine elektronische Vorrichtung umfassend eine Anode, eine Lochinjektionsschicht, eine Lochtransportschicht, eine emittierende Schicht, eine Elektronentransportschicht, eine Elektroneninjektionsschicht und eine Kathode, wobei eine Verbindung der Formel (II-1), bzw. Formel (II-2) bevorzugt in der emittierenden Schicht enthalten ist.

Bevorzugt umfasst der Gegenstand der Erfindung eine elektronische Vorrichtung umfassend eine Emissionsschicht, welche die Verbindung H1 oder H2 und eine Verbindung der Formel (II-1), bzw. Formel (II-2), bevorzugt eine Verbindung D1, D2, D3 oder D4 umfasst, und umfassend eine Elektronentransportschicht, welche die Verbindung ETL umfasst.

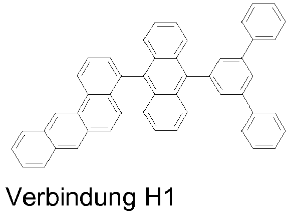

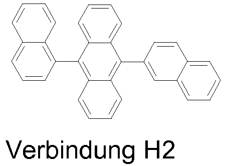

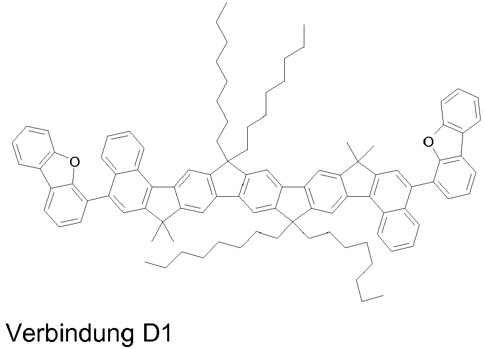

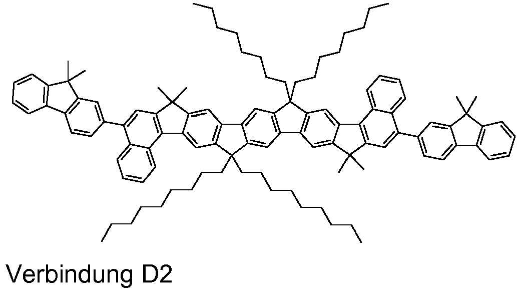

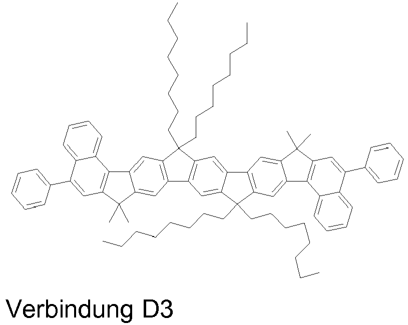

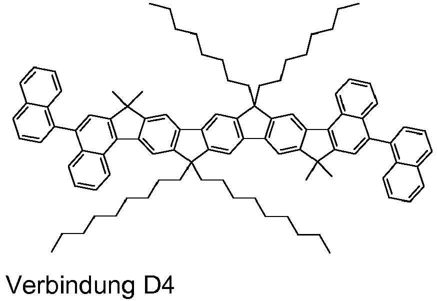

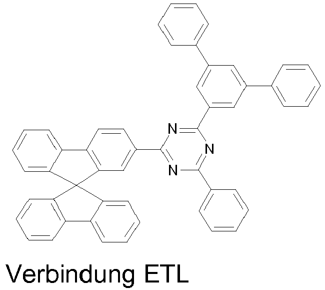

Die erfindungsgemäße organische Elektrolumineszenzvorrichtung enthält bevorzugt eine emittierende Schicht, welche ein Emissionsmaximum im blauen Farbbereich in einem Wellenlängenbereich zwischen 420 nm und 490 nm aufweist.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (II-1), bzw. Formel (II-2) enthält und wobei die drei Schichten blaue, grüne, gelbe, orangefarbene oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Alternativ und/oder zusätzlich können in einer derartigen organischen Elektrolumineszenzvorrichtung die erfindungsgemäßen Verbindungen auch in der Lochtransportschicht oder in einer anderen Schicht vorhanden sein. Die verschiedenen emittierenden Schichten können direkt aneinander grenzen, oder sie können durch nicht emittierende Schichten voneinander getrennt sein. Gemäß einer bevorzugten Ausführungsform der Erfindung ist eine weiß emittierende OLED eine sogenannte Tandem-OLED, das heißt es liegen zwei oder mehr vollständige OLED-Schichtabfolgen in der OLED vor, wobei die OLED-Schichtabfolgen jeweils Lochtransportschicht, emittierende Schicht und Elektronentransportschicht umfassen, die jeweils durch eine Ladungserzeugungsschicht (charge generation layer) voneinander getrennt sind.

Es ist bevorzugt, wenn die Verbindung gemäß Formel (II-1), bzw. Formel (II-2) in einer emittierenden Schicht eingesetzt wird. Insbesondere eignet sich die Verbindung gemäß Formel (II-1), bzw. Formel (II-2) zur Verwendung als emittierende Verbindung oder als Matrixmaterial in einer emittierenden Schicht.

Die erfindungsgemäße Verbindung eignet sich besonders zur Verwendung als blau emittierende Emitterverbindung oder als Matrixverbindung für eine blau emittierende Emitterverbindung. Dabei kann die betreffende elektronische Vorrichtung eine einzige emittierende Schicht enthaltend die erfindungsgemäße Verbindung enthalten, oder sie kann zwei oder mehr emittierende Schichten enthalten. Die weiteren emittierenden Schichten können dabei eine oder mehrere erfindungsgemäße Verbindungen oder alternativ andere Verbindungen enthalten.

Wenn die erfindungsgemäße Verbindung als Matrixmaterial in einer emittierenden Schicht einer OLED eingesetzt wird, ist es bevorzugt, dass keiner der Substituenten R¹, R² und R³ gewählt ist aus mit dem Grundgerüst der Formel (II-1), bzw. Formel (II-2) konjugierten Gruppen, insbesondere keiner der Substituenten R¹, R² und R³ gewählt ist aus Cyanogruppen, Arylaminogruppen oder Aryl- oder Heteroarylgruppen. Besonders bevorzugt sind bei Verwendung der erfindungsgemäßen Verbindung als Matrixmaterial R¹ und R² gewählt aus H, D, F und Alkylgruppen mit 1 bis 10 C-Atomen, besonders bevorzugt aus H und D, ganz besonders bevorzugt sind R¹ und R² gleich H.

Wenn die erfindungsgemäße Verbindung als Emitterverbindung in einer emittierenden Schicht einer OLED eingesetzt wird, ist es bevorzugt, dass ein oder mehrere Substituenten R¹, R² und R³ gewählt sind aus mit dem Grundgerüst der Formel (II-1), bzw. Formel (II-2)) konjugierten Gruppen, beispielsweise Cyanogruppen, Arylaminogruppen oder Aryl- oder Heteroarylgruppen.

Wenn die erfindungsgemäße Verbindung als emittierende Verbindung in einer emittierenden Schicht eingesetzt wird, wird sie bevorzugt in Kombination mit einem oder mehreren Matrixmaterialien eingesetzt. Unter einem Matrixmaterial wird dabei ein Material verstanden, welches in der emittierenden Schicht vorliegt, bevorzugt als Hauptkomponente, und welches im Betrieb der Vorrichtung nicht Licht emittiert.

Der Anteil der emittierenden Verbindung in der Mischung der emittierenden Schicht beträgt zwischen 0.1 und 50.0 %, bevorzugt zwischen 0.5 und 20.0 %, besonders bevorzugt zwischen 1.0 und 10.0 -%. Entsprechend beträgt der Anteil des Matrixmaterials bzw. der Matrixmaterialien zwischen 50.0 und 99.9 %, bevorzugt zwischen 80.0 und 99.5 %, besonders bevorzugt zwischen 90.0 und 99.0 %.

Dabei wird unter den Angaben der Anteile in % im Rahmen der vorliegenden Anmeldung Vol.-% verstanden, wenn die Verbindungen aus der Gasphase aufgebracht werden, und es wird darunter Gew.-% verstanden, wenn die Verbindungen aus Lösung aufgebracht werden.

Wird die erfindungsgemäße Verbindung als Matrixmaterial eingesetzt, kann sie mit allen bekannten emittierenden Verbindungen kombiniert eingesetzt werden. Bevorzugt wird sie in Kombination mit den unten angegebenen bevorzugten emittierenden Verbindungen eingesetzt, besonders den unten angegebenen bevorzugten fluoreszierenden Verbindungen.

Wenn die Verbindung der Formel (II-1), bzw. Formel (II-2) als Matrixmaterial in Kombination mit einem phosphoreszierenden Emitter in einer emittierenden Schicht eingesetzt wird, ist der phosphoreszierende Emitter bevorzugt ausgewählt aus den unten aufgeführten Klassen und Ausführungsformen von phosphoreszierenden Emittern. Weiterhin sind in diesem Fall in der emittierenden Schicht bevorzugt ein oder mehrere weitere Matrixmaterialien vorhanden.

Solche sogenannten Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Bevorzugt stellt die Verbindung der Formel (II-1), bzw. Formel (II-2) das Material mit lochtransportierenden Eigenschaften dar.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im Mixed-Matrix-System eingesetzt wird.

Die erfindungsgemäßen Verbindungen können auch in anderen Schichten eingesetzt werden, beispielsweise als Lochtransportmaterialien in einer Lochinjektions- oder Lochtransportschicht oder Elektronenblockierschicht.

Wird die Verbindung gemäß Formel (II-1), bzw. Formel (II-2) als Lochtransportmaterial eingesetzt, beispielsweise in einer Lochtransportschicht, einer Lochinjektionsschicht oder einer Elektronenblockierschicht, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (II-1), bzw. Formel (II-2) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Weiterhin ist es in diesem Fall bevorzugt, dass die elektronische Vorrichtung mehrere lochtransportierende Schichten zwischen Anode und emittierender Schicht aufweist. Es kann der Fall auftreten, dass alle diese Schichten eine Verbindung der Formel (II-1), bzw. Formel (II-2)) enthalten, oder dass nur einzelne dieser Schichten eine Verbindung der Formel (II-1), bzw. Formel (II-2) enthalten.

Wird die Verbindung der Formel (II-1), bzw. Formel (II-2) als Lochtransportmaterial eingesetzt, so ist es bevorzugt, dass sie einen großen Abstand zwischen dem HOMO- und dem LUMO-Energieniveau aufweist. Weiterhin ist es bevorzugt, dass sie keine Aminogruppen als Substituenten aufweist. Weiterhin ist es bevorzugt, dass sie überhaupt keine Substituenten an den aromatischen Ringen aufweist, d.h. dass R¹ und R² gleich H oder D, besonders bevorzugt gleich H sind.

Die Verbindung der Formel (II-1), bzw. Formel (II-2) kann weiterhin als elektronentransportierende Verbindung in einer Elektronentransportschicht, einer Lochblockierschicht oder einer Elektroneninjektionsschicht eingesetzt werden. Hierfür ist es bevorzugt, dass die Verbindung der Formel (II-1), bzw. Formel (II-2) einen oder mehrere Substituenten gewählt aus elektronenarmen Heteroarylgruppen wie beispielsweise Triazin, Pyrimidin oder Benzimidazol enthält.

Im Folgenden sind allgemein bevorzugte Materialklassen zur Verwendung als entsprechende Funktionsmaterialien in den erfindungsgemäßen organischen Elektrolumineszenzvorrichtungen aufgeführt.

Als phosphoreszierende emittierende Verbindungen eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Emitter können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Emitter sind neben den erfindungsgemäßen Verbindungen ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und WO 2013/185871 offenbarten Pyren-Arylamine. Ebenfalls bevorzugt sind die in WO 2014/037077 offenbarten Benzoindenofluoren-Amine, die in der noch nicht offengelegten EP 13000012.8 offenbarten Benzofluoren-Amine und die in der noch nicht offengelegten EP13004921.6 offenbarten erweiterten Indenofluorene.

Bevorzugte fluoreszierende emittierende Verbindungen sind in der folgenden Tabelle abgebildet:

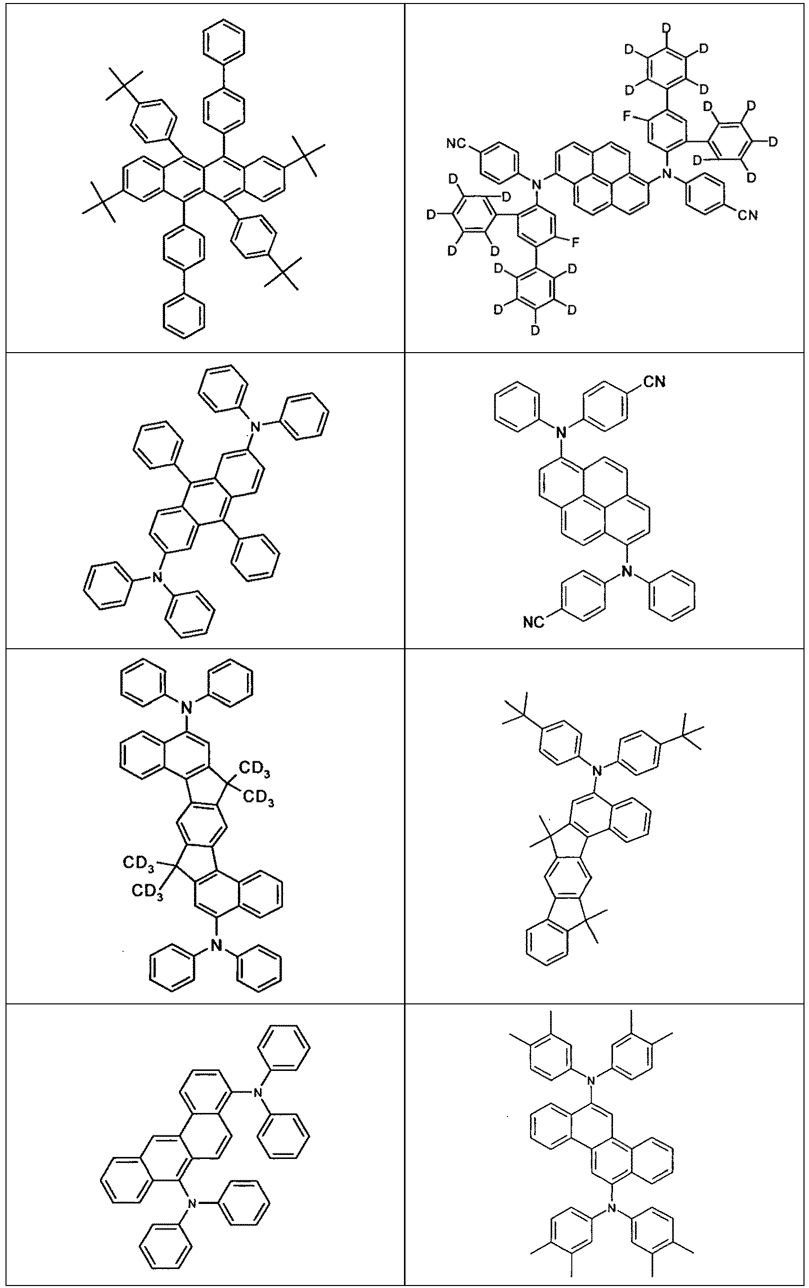

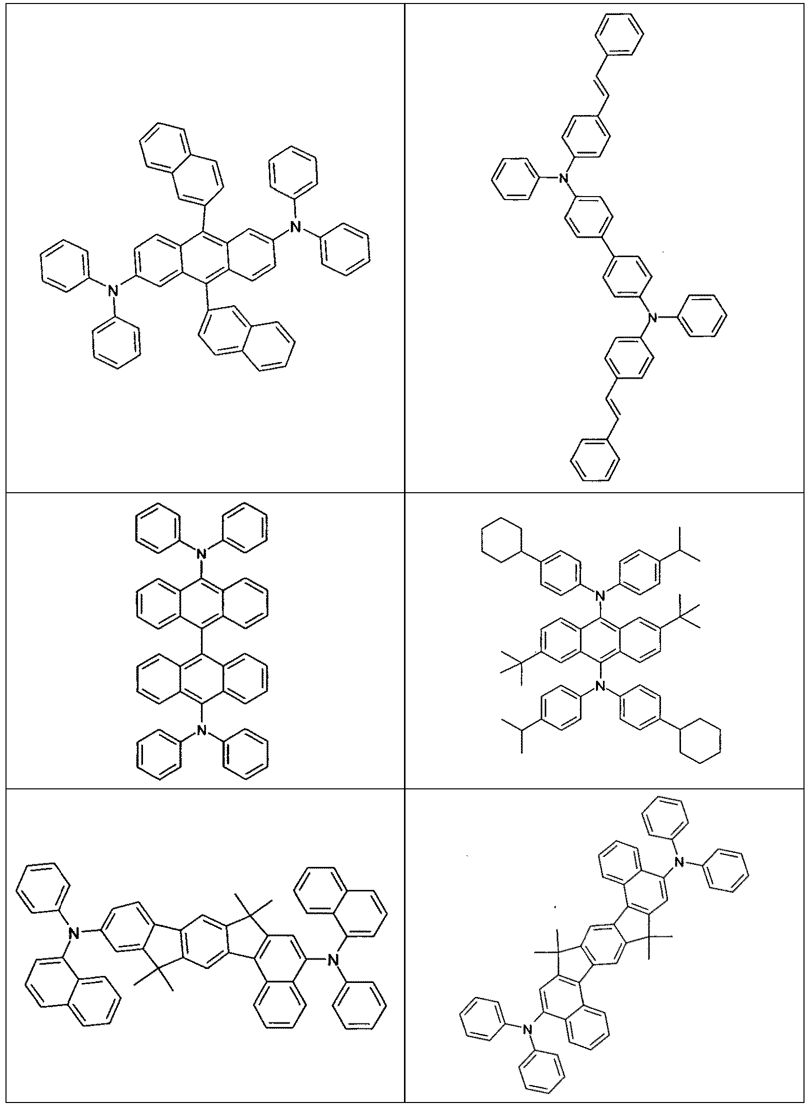

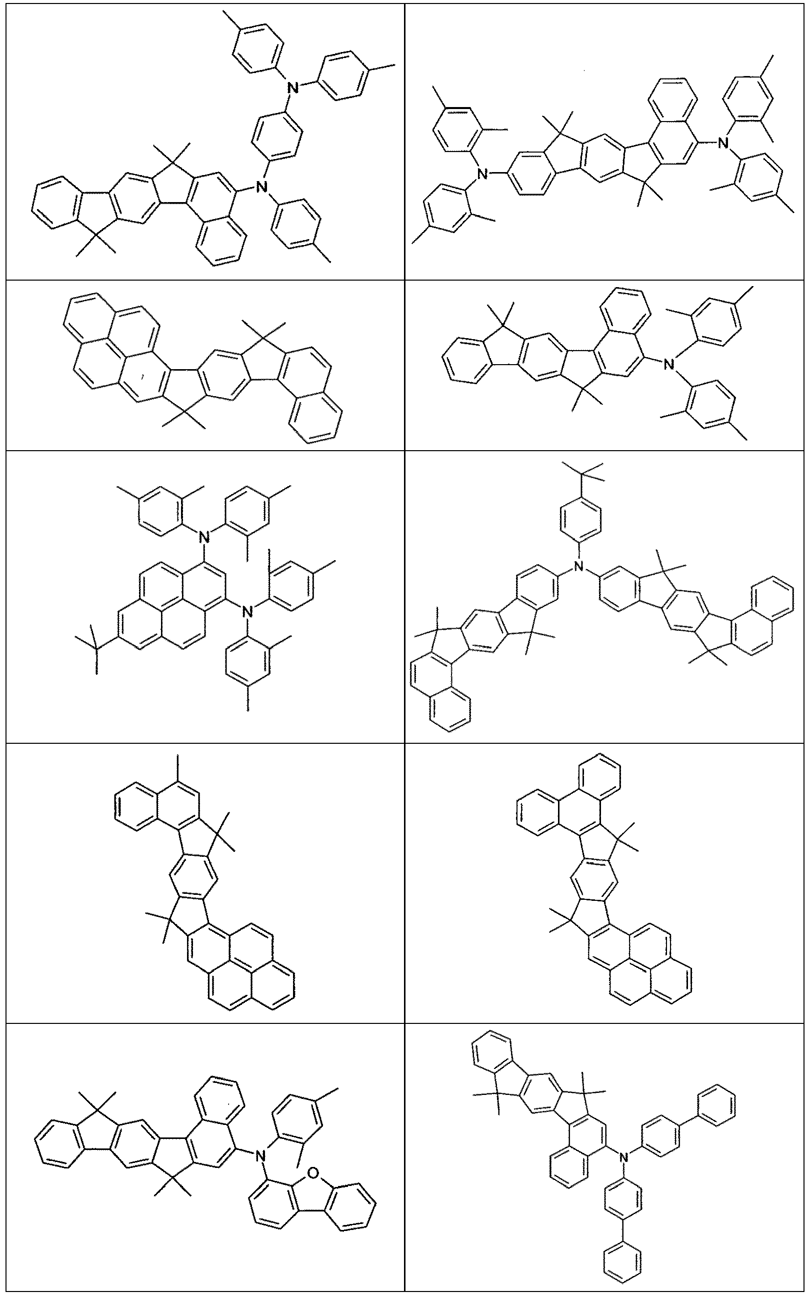

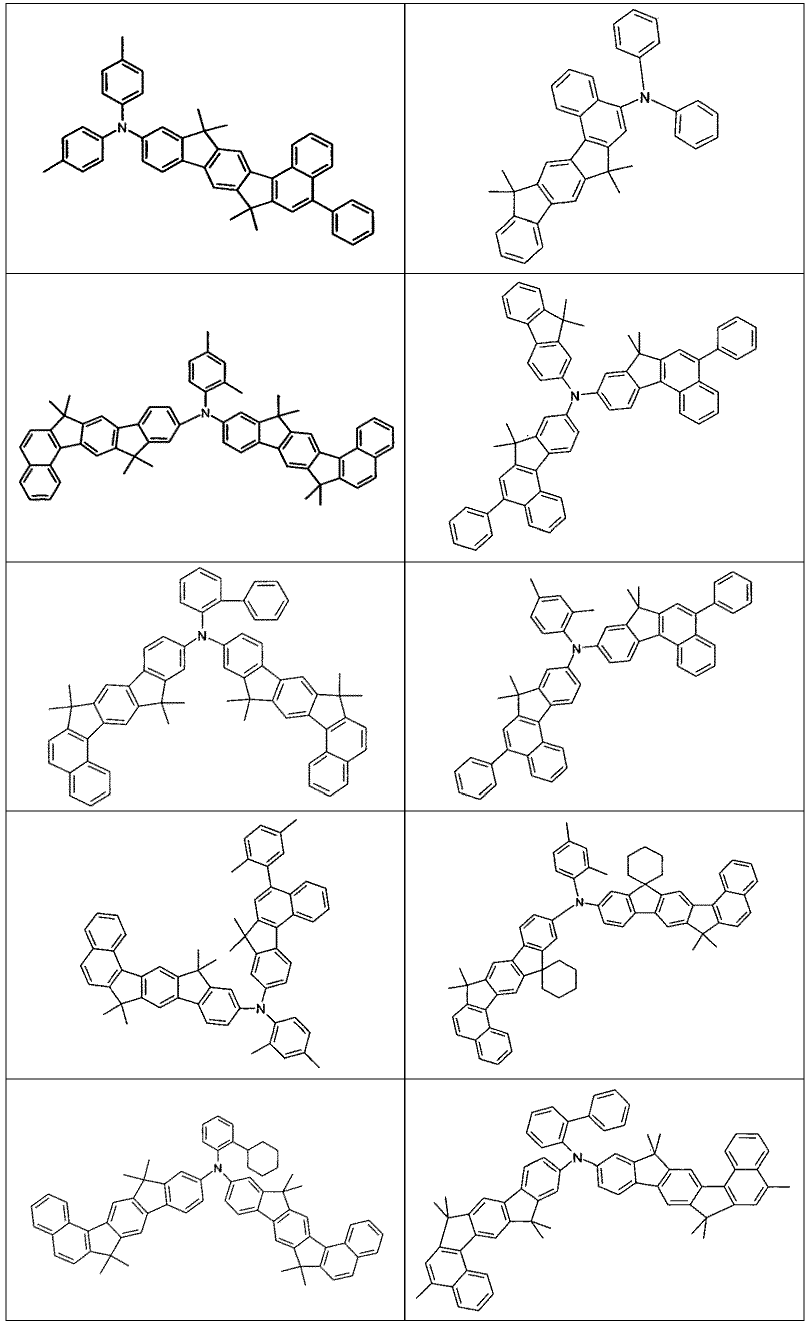

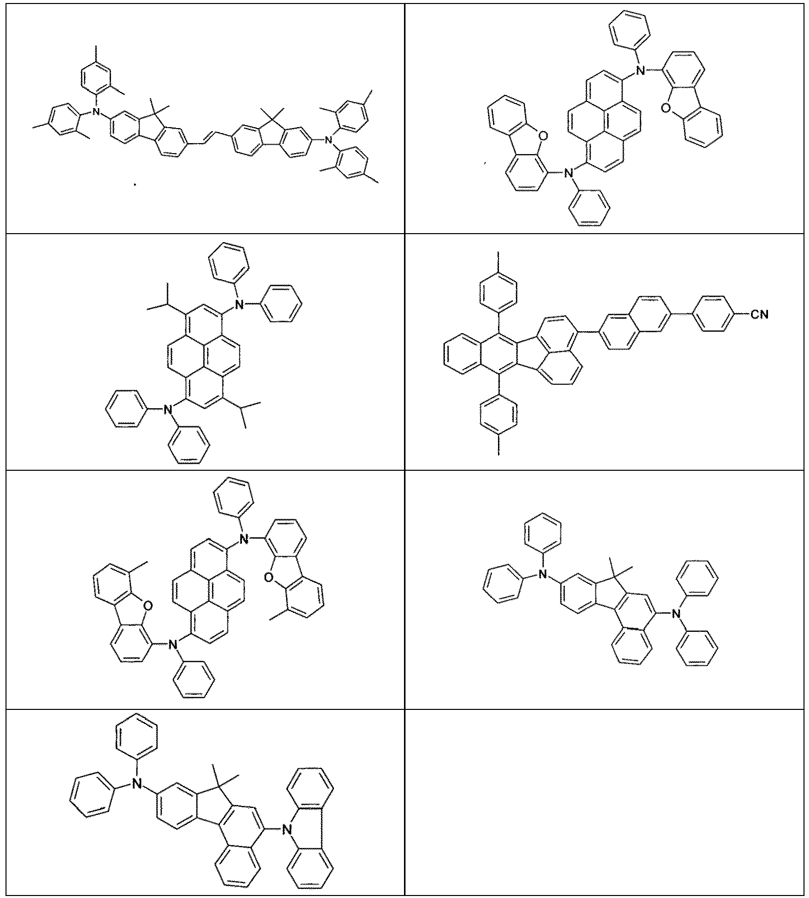

Bevorzugte Matrixmaterialien zur Verwendung in Kombination mit den erfindungsgemäßen Verbindungen als Emitter sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien zur Verwendung in Kombination mit der Verbindung der Formel (II-1), bzw. Formel (II-2) in der emittierenden Schicht sind in der folgenden Tabelle abgebildet.

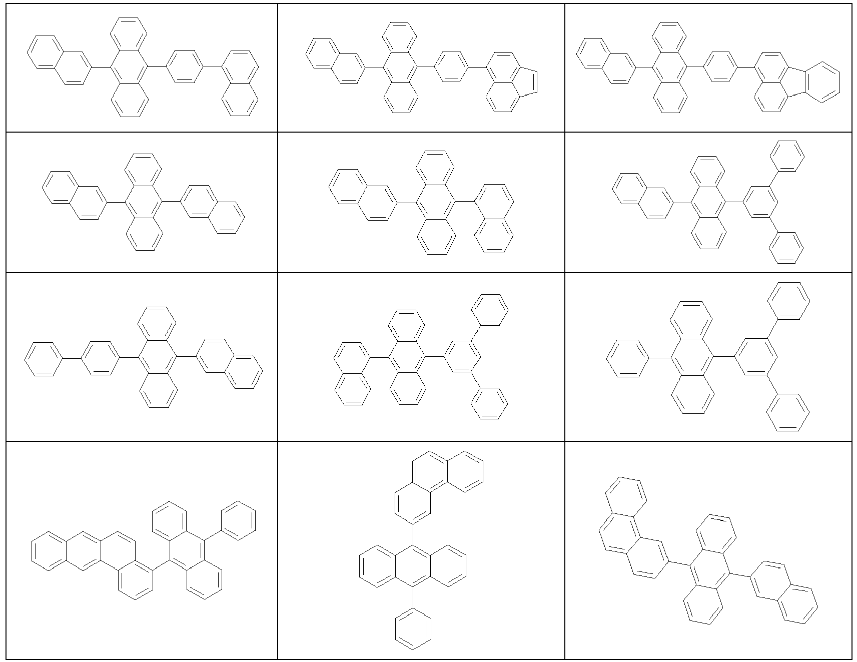

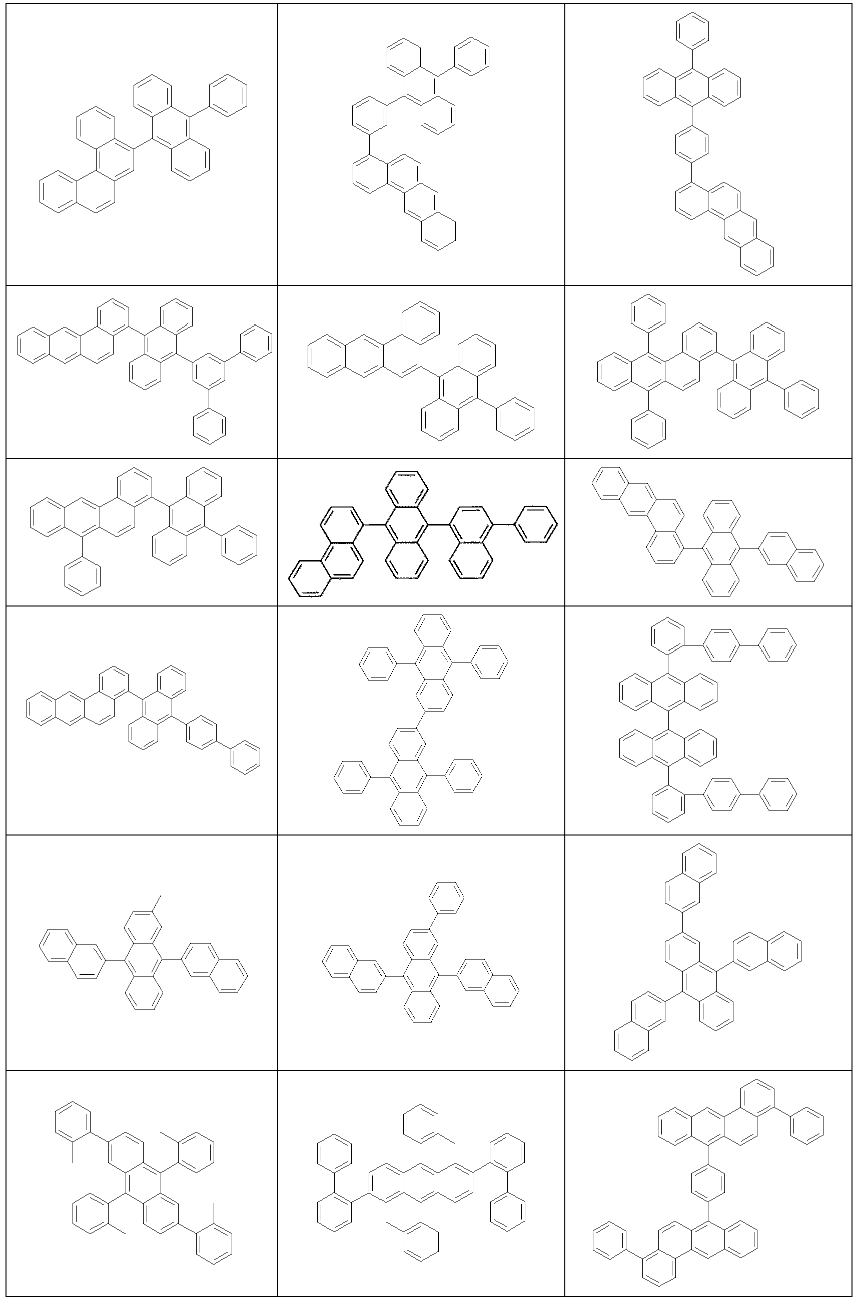

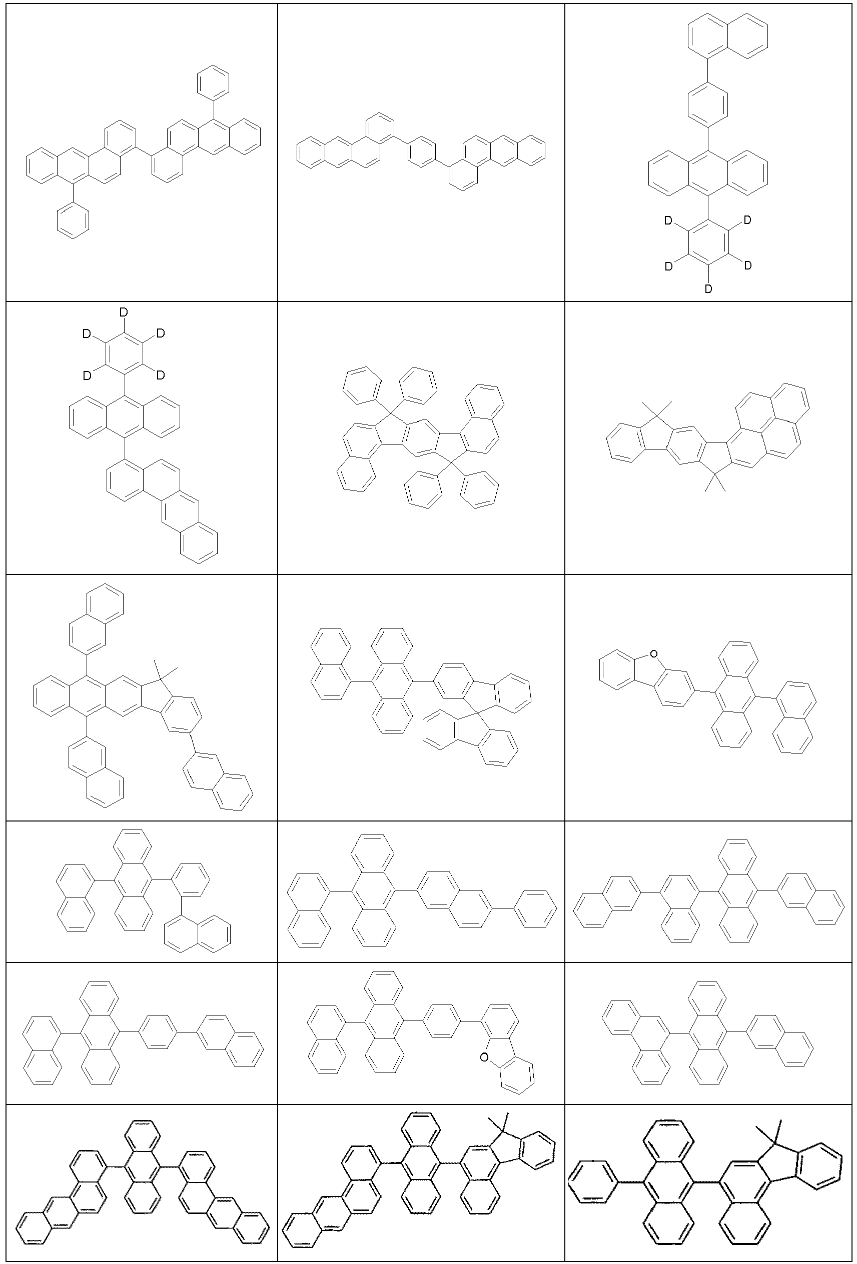

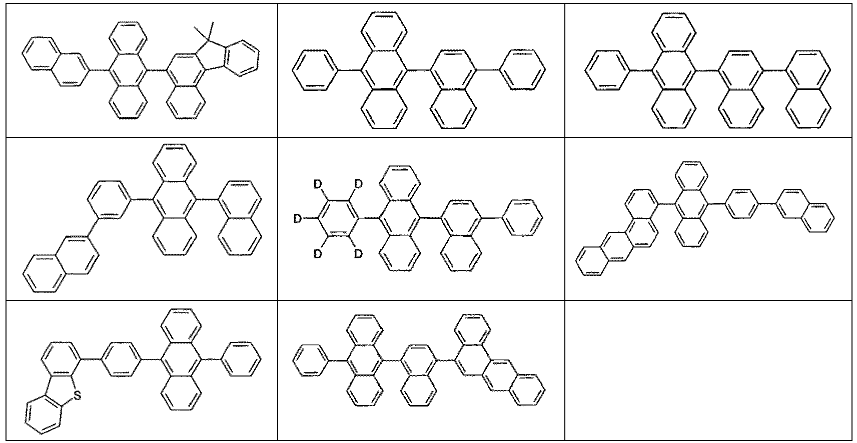

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport-, Lochinjektions- oder Elektronenblockierschicht in der erfindungsgemäßen Elektrolumineszenzvorrichtung verwendet werden können, sind neben den Verbindungen der Formel (II-1), bzw. Formel (II-2) Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001).

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z.B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, CS₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer als 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck geringer als 10⁻⁵ mbar, bevorzugt geringer als 10⁻⁶ mbar aufgedampft. Dabei ist es jedoch auch möglich, dass der Anfangsdruck noch geringer ist, beispielsweise geringer als 10⁻⁷ mbar ist.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (II-1), bzw. Formel (II-2) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

Es wird nach folgendem allgemeinen Schema vorgegangen:

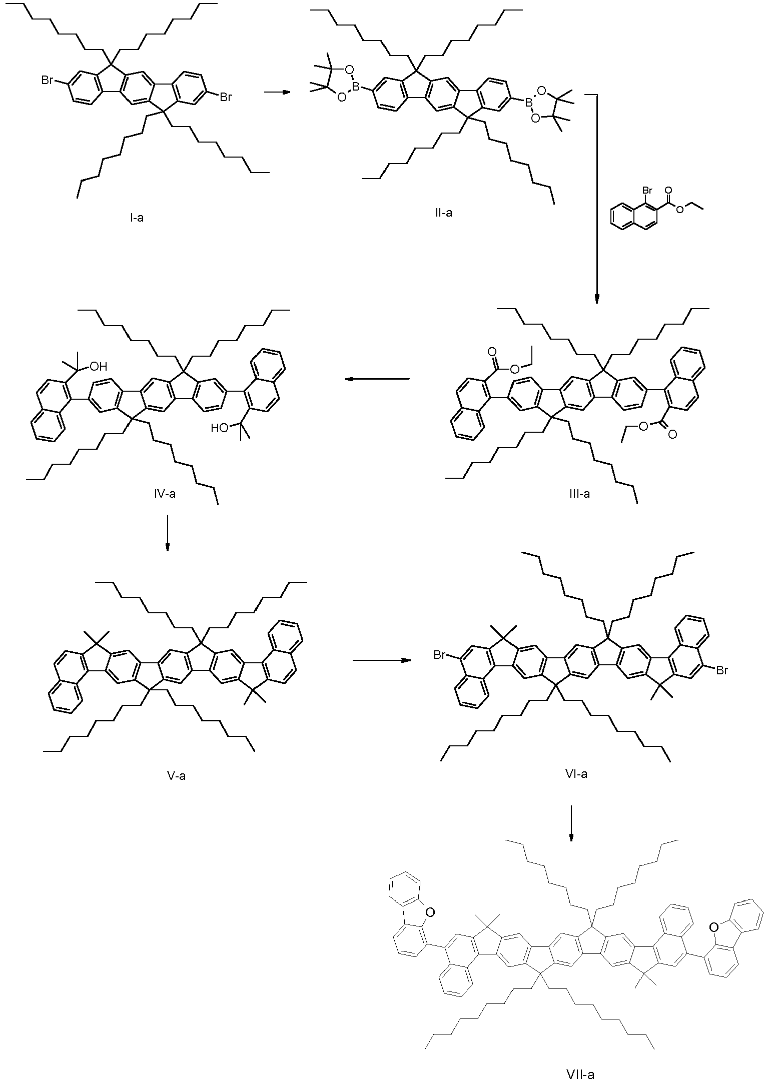

Analog dazu wird von folgender Verbindung ausgegangen:

| **Verbindung** | 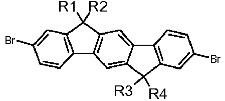 |
|---|---|
| **I-b** | 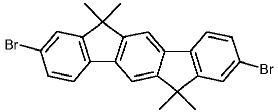 |

### Verbindung II-a

I-a, 2,8-Dibromo-6,12-dihydro-6,6,12,12-tetraoctyl-indeno[1,2-b]fluoren, (100 g, 116 mmol), Bis-(pinacolato)-diboran (64.9 g, 256 mmol) und Kaliumacetat (75.2 g, 767 mmol) werden in 1 L Tetrahydrofuran suspendiert. Die Lösung wird entgast und mit Argon gesättigt. Danach wird PdCl₂(dppf)-CH₂Cl₂ (1.9 g, 2.3 mmol) zugegeben. Die Reaktionsmischung wird für 16 h unter Schutzgasatmosphäre zum Sieden erhitzt, anschließend auf Raumtemperatur abgekühlt und unter vermindertem Druck eingeengt. Der Feststoff wird mit einer Mischung aus Dichlormethan und Wasser versetzt und ausgeschüttelt. Die Phasen werden getrennt und die wässrige Phase wird zweimal mit Dichlormethan extrahiert. Die vereinigten organischen Phasen werden mit Wasser gewaschen, über Natriumsulfat getrocknet, filtriert und unter vermindertem Druck eingeengt. Das Rohprodukt wird mit Toluol über SiO₂ / Al₂O₃ filtriert und anschließend das Lösungsmittel im Vakuum entfernt. Der verbleibende Rückstand wird mit Methanol ausgerührt und dann filtriert. Die Ausbeute beträgt 94.5 g (81% d. Th., Reinheit ca. 95 %).

Analog dazu wird die folgende Verbindung hergestellt:

| **Verbindung** | 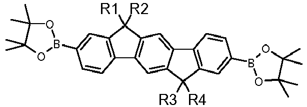 | **Ausbeute** |
|---|---|---|
| **II-b** | 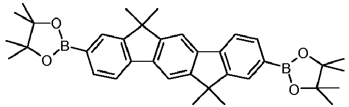 | 71 % |

### Verbindung III-a

II-a (85 g, 84.6 mmol), 1-Brom-naphthalin-2-ethylester (54.3 g, 194 mmol) und Natriumcarbonat (32.5 g, 306 mmol) werden einem Gemisch aus Wasser/Toluol/Ethanol (Verhältnis 1:2:1, 3 L) suspendiert. Die Lösung wird entgast und mit Argon gesättigt. Danach wird Tetrakis(triphenylphosphin)-palladium(0) (1.95 g, 1.7 mmol) zugegeben. Die Reaktionsmischung wird für 16 h unter Schutzgasatmosphäre zum Sieden erhitzt. Die Phasen werden getrennt und die wässrige Phase wird zweimal mit Toluol extrahiert. Die vereinigten organischen Phasen werden mit Wasser gewaschen, über Natriumsulfat getrocknet, filtriert und anschließend unter vermindertem Druck eingeengt. Das Gemisch wird über Celite mit Toluol filtriert und das Lösungsmittel anschließend unter vermindertem Druck entfernt. Der verbleibende Rückstand wird mit Methanol ausgerührt und anschließend filtriert. Die Ausbeute beträgt 89.0 g (96 % d. Th.).

Analog dazu wird folgende Verbindung hergestellt:

| **Verbindung** | 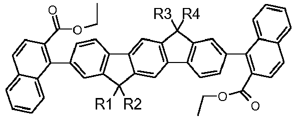 | **Ausbeute** |
|---|---|---|
| **III-b** | 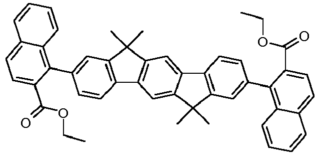 | 73 % |

### Verbindung IV-a

Zu III-a (89 g, 81 mmol) in 1 L wasserfreiem Tetrahydrofuran wird in eine Mischung aus wasserfreiem Ceriumchlorid (41.9 g, 170 mmol) und 500 mL wasserfreiem Tetrahydrofuran bei einer Temperatur zwischen 0 °C und 5 °C zugetropft. Die Reaktionsmischung wird für 1 h bei dieser Temperatur gerührt. Anschließend werden 400 mL gesättigte wässrige Ammoniumchlorid-Lösung bei einer Temperatur zwischen 0 °C und 20 °C zugetropft. Die erhaltene Suspension wird filtriert. Die Phasen werden anschließend getrennt und die wässrige Phase wird zweimal mit 200 mL Ethylacetat extrahiert. Die vereinigten organischen Phasen werden unter reduziertem Druck eingeengt. Die Ausbeute beträgt 59.8 g (69 % d. Th.).

Analog dazu wird folgende Verbindung hergestellt:

| **Verbindung** | 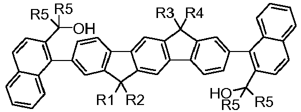 | **Ausbeute** |
|---|---|---|
| **IV-b** | 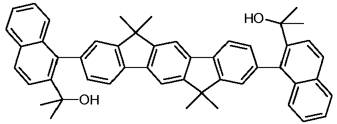 | 48 % |

### Verbindung V-a

Methansulfonsäure (10.8 mL, 167 mmol) wird zu einer Mischung aus Polyphosphorsäure (16.4 g, 167 mmol) und 700 mL Dichlormethan bei einer Temperatur von 0 °C zugetropft. Anschließend wird eine Suspension aus IV-a (59.8 g, 56 mmol) in 800 mL Dichlormethan langsam bei 0 °C zugetropft. Die Reaktionsmischung wird 2 h bei 0 °C gerührt. 800 mL Ethanol werden zugegeben und die Reaktionsmischung wird anschließend für 30 min gerührt. Das Lösungsmittel wird unter reduziertem Druck entfernt und der verbleibende Rückstand wird zweimal mit einer Mischung aus Toluol und Heptan umkristallisiert. Die Ausbeute beträgt 46.3 g (80 % d. Th.).

Analog dazu wird folgende Verbindung hergestellt:

| | | |
|---|---|---|
| **Verbindung** | 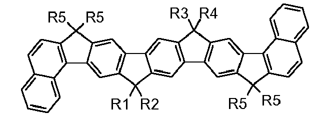 | **Ausbeute** |
| **V-b** | 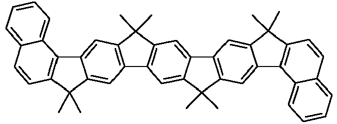 | **39 %** |

### Verbindung VI-a

V-a (41.3 mg, 40 mmol) wird in 1 L Dichlormethan gelöst. Anschließend wird bei 0 °C Br₂ (12.74 g, 79.7 mmol) in 300 mL Dichlormethan zugetropft. Die Reaktionsmischung wird über Nacht bei Raumtemperatur gerührt. 200 mL Natriumthiosulfat-Lösung wird zugegeben und die Reaktionsmischung wird für 30 min gerührt. Anschließend werden die Phasen getrennt. Die organische Phase wird mit Wasser gewaschen, über Natriumsulfat getrocknet und einrotiert. Die Reaktionsmischung wird über SiO₂ und Al₂O₃ mit Toluol filtriert und einrotiert. Der verbleibende Rückstand wird zweimal in Toluol/Heptan umkristallisiert. Die Ausbeute beträgt 40.8 g (86 % d. Th.).

### Verbindung VII-a

VI-a (20 g, 16.5 mmol), Dibenzofuran-4-ylboronsäure (7.7 g, 36.3 mmol) und Trikaliumphosphat-Monohydrat (15.2 g, 66 mmol) werden in einem Gemisch aus Toluol/Dioxan/ Wasser (1:1:1, 600 mL) suspendiert. Anschließend werden Palladiumacetat (74 mg, 0.33 mol) und Tri(o-tolyl)-phosphin (602 mg, 2.0 mmol) zugegeben. Die Reaktionsmischung wird für 16 h zum Sieden erhitzt. Nach dem Abkühlen der Reaktionsmischung auf Raumtemperatur wird die organische Phase mit 300 mL Ethylacetat erweitert. Die Phasen werden getrennt und die wässrige Phase wird zweimal mit Ethylacetat extrahiert. Die vereinigten organischen Phasen werden über Natriumsulfat getrocknet und anschließend unter reduziertem Druck eingeengt. Das Gemisch wird über Aluminiumoxid mit Toluol filtriert und der verbleibende Rückstand wird anschließend mehrmals in Toluol/Heptan umkristallisiert. Die Ausbeute beträgt 18.3 g (82 % d. Th.).

Analog dazu werden die folgenden Verbindungen hergestellt:

| **Verbindung** | 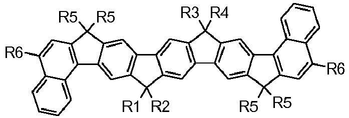 | **Ausbeute** |
|---|---|---|
| **VII-b** | 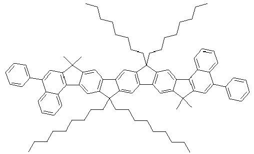 | 73 % |
| **VII-c** | 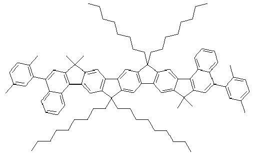 | 64 % |
| **VII-d** | 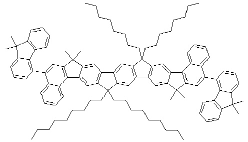 | 37 % |
| **VII-e** | 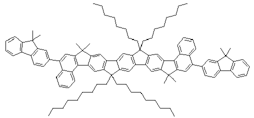 | 48 % |
| **VII-f** | 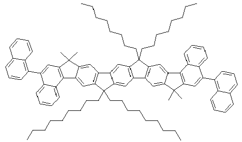 | 54 % |
| **VII-g** | 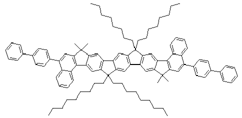 | 61 % |

### B) Emissionsdaten

Die Verbindung der Formel (II-1), bzw. Formel (II-2) wird in Toluol gelöst und daraufhin Absorptionsspektren und/oder Photolumineszenzspektren der entsprechenden Verbindung der Formel (II-1), bzw. Formel (II-2) aufgenommen.

Absorptionsspektren wurden an dem Gerät Lambda 9, UV/VIS/NIR Spectrometer, der Firma Perkin Elmer gemessen.

Photolumineszenzspektren wurden an dem Gerät F-4500, Flourescence Spectrophotometer, der Firma Hitachi gemessen.

### B-1) Emissionsdaten der Vergleichsverbindung (1)

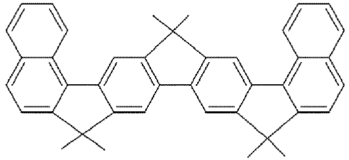

Vergleichsverbindung (1)

| | **Maxima** [nm]³ |
|---|---|
| **Absorption¹** | 362 |
| | 380 |
| | 402 |
| **Emission²** | 409 |
| | 432 |
| | 458 |

### B-2) Emissionsdaten der Zielverbindung (1)

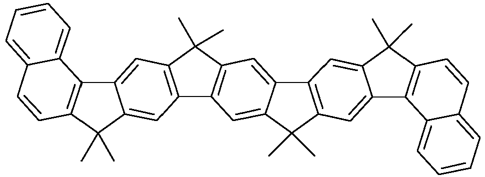

Zielverbindung (1)

| | **Maxima** [nm]³ |
|---|---|
| **Absorption¹** | 371 |
| | 391 |
| | 414 |
| **Emission²** | 420 |
| | 445 |
| | 473 |

| | |
|---|---|
| ¹: Absorptionsmessung ²: Emissionsmessung ³: Maxima der Absorptions,- bzw. Emissionspeaks in nm, das Hauptmaximum in nm ist unterstrichen | |

Bei Anregung emittiert die Vergleichsverbindung (1), welche auf einem Benzindenofluoren-Grundkörper basiert, ultraviolettes und violettes Licht mit Wellenlängen von 409 nm, 432 nm und 458 nm.

Überraschend wurde nun gefunden, dass die erfindungsgemäßen Verbindungen der Formel (II-1)bzw. Formel (II-2), welche auf einem erweiterten Bis-Indenofluoren-Grundgerüst basieren, Emissionen aufweisen, welche zu größeren Wellenlängen verschoben sind. Die Zielverbindung (1) weist insbesondere Emissionen auf, die teilweise im Wellenlängenbereich des blauen sichtbaren Licht liegen. So zeigt die Zielverbindung 1 Emissionspeaks bei 420 nm, 445 nm und 473 nm.

Somit weist die Zielverbindung (1) bei Anregung eine blaue Emission auf und eignet sich dementsprechend für die Verwendung als blauer Singulett-Emitter in elektronischen Vorrichtungen.

### C) Device-Beispiele: Herstellung der OLEDs

Die Herstellung von erfindungsgemäßen OLEDs sowie OLEDs nach dem Stand der Technik erfolgt nach einem allgemeinen Verfahren gemäß WO 2004/058911. Die Herstellung lösungsbasierter OLEDs ist z.B. in der WO 2004/037887 und in der WO 2010/097155 beschrieben. Bei den folgenden Beispielen wurden beide Herstellungsverfahren kombiniert, so dass bis einschließlich der Emissionsschicht der OLED aus Lösung prozessiert wird und die darauffolgenden Schichten (z.B. Elektronentransportschicht der OLED) im Vakuum aufgedampft werden. Die vorbeschriebenen allgemeinen Verfahren werden wie folgt kombiniert und auf die hier beschriebenen Gegebenheiten (Variation der Schichtdicken, Materialien) angepasst.

In den folgenden Beispielen (siehe Tabelle 1 und 2) werden die Daten verschiedener OLEDs vorgestellt. Als Substrate werden Glassubstrate verwendet, die mit strukturiertem ITO (Indium Zinn Oxid) der Dicke 50 nm beschichtet sind. Die OLEDs haben prinzipiell folgenden Schichtaufbau: Substrat / Buffer (20 nm) / Lochtransportschicht (HTL, 20 nm) / Emissionsschicht (EML, 60 nm) / Elektronentransportschicht (ETL, 20 nm) / Elektroneninjektionsschicht (EIL, 3 nm) und abschließend eine Kathode. Die Kathode wird durch eine 100 nm dicke Aluminiumschicht gebildet. Das Substrat wird mit einem Buffer aus Pedot:PSS (Poly(3,4-ethylendioxy-2,5-thiophen) : Polystyrolsulfonat), bezogen von Heraeus Precious Metals GmbH & Co. KG, beschichtet. Das Aufschleudern erfolgt an Luft aus Wasser. Die Schicht wird anschließend für 10 Minuten bei 180 °C ausgeheizt. Auf die so beschichteten Substrate werden die Lochtransportschicht, sowie die Emissionsschicht aufgebracht. Die Strukturen der in der OLED verwendeten Materialien sind in Tabelle 2 gezeigt, wobei HTL das Material der Lochtransportschicht, wobei EIL das Material der Elektroneninjektionsschicht, und wobei ETL das Material der Elektronentransportschicht darstellt.

Die Lochtransportschicht besteht aus dem Polymer HTL, der in der Tabelle 2 gezeigten Struktur, das gemäß WO 2010/097155 synthetisiert wurde. Das Polymer wird in Toluol gelöst, so dass die Lösung einen Feststoffgehalt von ca. 5 g/l besitzt, um eine Schichtdicke von 20 nm mittels Spincoating zu erzielen. Die Schichten werden in einer Atmosphäre aus inertem Gas, im vorliegenden Fall Argon, aufgeschleudert und für 60 min bei 180 °C ausgeheizt.

Die Emissionsschicht (EML) besteht immer aus mindestens einem Matrixmaterial (Host=H) und einer emittierenden Verbindung (Emitter, Dotand=D), die dem Matrixmaterial in einem bestimmten Gewichtsanteil beigemischt wird. Eine Angabe wie H1:D1 (92%:8%) bedeutet hierbei, dass das Matrixmaterial H1 in einem Gewichtsanteil von 92% und die emittierende Verbindung D1 in einem Gewichtsanteil von 8% in der Emissionsschicht vorliegt. Die Mischung für die Emissionsschicht wird in Toluol gelöst, so dass die Lösung einen Feststoffgehalt von ca. 18 g/l aufweist, um eine Schichtdicke von 60 nm mittels Spincoating zu erzielen. Die Schichten werden in einer Atmosphäre aus inertem Gas, im vorliegenden Fall Argon, aufgeschleudert und für 10 min bei 140 °C ausgeheizt. Die verwendeten Matrixmaterialien H und die Dotanden D sind in Tabelle 1 gezeigt. Die Strukturen der in der Emissionsschicht der OLED verwendeten Materialien sind in Tabelle 2 gezeigt.

Die Materialien für die Elektronentransportschicht sowie für die Kathode werden in einer Vakuumkammer thermisch aufgedampft. Dabei kann z.B. die Elektronentransportschicht aus mehr als einem Material bestehen, die einander durch Co-Verdampfung in einem bestimmten Volumenanteil beigemischt werden. Eine Angabe wie ETM:EIL (50%:50%) bedeutet hierbei, dass die Materialien ETM und EIL in einem Volumenanteil von je 50% in der Schicht vorliegen. Im vorliegenden Fall besteht die Elektronentransportschicht aus dem Material ETL mit einer Schichtdicke von 20 nm und die Elektroneninjektionsschicht besteht aus dem Materials EIL mit einer Schichtdicke von 3 nm. Das Material ETL und das Material EIL sind in Tabelle 2 gezeigt.

Die OLEDs werden standardmäßig charakterisiert. Hierfür werden die Elektrolumineszenzspektren aufgenommen, die Stromeffizienz (gemessen in cd/A) und die externe Quanteneffizienz (EQE, gemessen in Prozent) in Abhängigkeit der Leuchtdichte unter Annahme einer lambertschen Abstrahlcharakteristik aus Strom-Spannungs-Leuchtdichte-Kennlinien (IUL-Kennlinien) berechnet und abschließend die Lebensdauer der Bauteile bestimmt. Die Elektrolumineszenzspektren werden bei einer Leuchtdichte von 1000 cd/m² aufgenommen und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². Die Lebensdauer LD80 @ 10mA/cm² ist die Zeit, die vergeht, bis die Starthelligkeit bei einer Betriebsstromdichte von 10mA/cm² um 20% gesunken ist. Die erhaltenen Daten für die verschiedenen OLEDs sind in Tabelle 1 zusammengefasst.

### D) Verwendung der erfindungsgemäßen Verbindungen als Emitter in fluoreszierenden OLEDs

Es werden die erfindungsgemäßen Verbindungen D1, D2, D3 und D4 einzeln als Emitter in der emittierenden Schicht von OLEDs eingesetzt (Struktur, siehe Tabelle 2). Als Matrixmaterial der emittierenden Schicht wird dabei die Verbindung H1, bzw. H2, eingesetzt. Die erhaltenen OLEDs sind E1 bis E6. Sie zeigen sehr gute Lebensdauer (LD80) bei tiefblauer Emission (Tabelle 1). Verglichen mit im Stand der Technik bekannten Emittermaterialien (V-D1 und V-D2, vgl. V1 bis V3) ist die Quanteneffizienz verbessert und ist die Lebensdauer (LD80) deutlich verbessert.

Insbesondere der Vergleich mit dem Material V-D2 zeigt die Verbesserung, die durch das erfindungsgemäße erweiterte Bis-Indenofluoren-Grundgerüst gegenüber dem im Stand der Technik bekannten Bis-Indenofluoren-Grundgerüst erzielt wird.

| **Tabelle 1: Daten der OLEDs** | | | | | | |
|---|---|---|---|---|---|---|
| ***Beispiel*** | ***Host*** | ***Dotand*** | ***EQE* @ *1000 cd*/*m²*** | ***LD80* @ *10mA*/*cm²*** | ***CIE*** | |
| | *92 %* | *8 %* | *%* | *[h]* | *x* | *y* |
| *V1* | *H1* | *V-01* | *3.1* | *140* | 0.142 | 0.102 |
| *V2* | *H2* | *V-D1* | *3.1* | *160* | 0.141 | 0.108 |
| *V3* | *H1* | *V-D2* | *2.9* | *140* | 0.144 | 0.132 |
| *E1* | *H1* | *D1* | *3.9* | *220* | 0.142 | 0.116 |
| *E2* | *H2* | *D1* | *4.1* | *250* | 0.141 | 0.121 |
| *E3* | *H1* | *D2* | *3.8* | *290* | 0.131 | 0.150 |
| *E4* | *H1* | *D3* | *3.8* | *180* | 0.157 | 0.098 |
| *E5* | *H2* | *D3* | *3.9* | *200* | 0.152 | 0.101 |
| *E6* | *H1* | *D4* | *4.2* | *290* | 0.145 | 0.120 |

| **Tabelle 2: Strukturen der verwendeten Materialien** | |
|---|---|
| 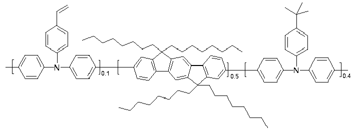 | 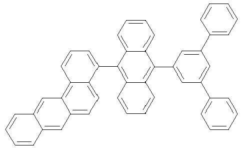 |
| HTL | H1 |
| 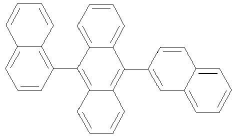 | 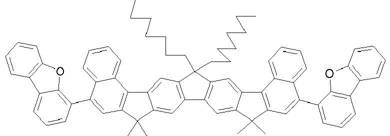 |
| H2 | V-D1 |
| 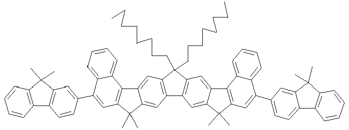 | 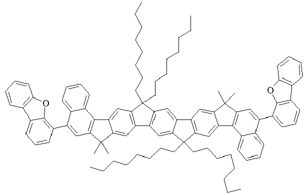 |
| V-D2 | D1 |
| 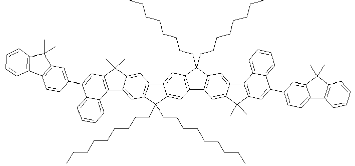 | 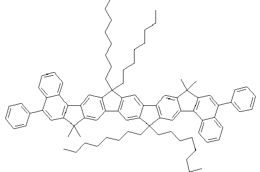 |
| D2 | D3 |
| 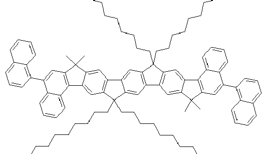 | 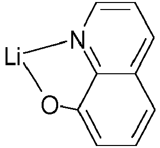 |
| D4 | EIL |
| 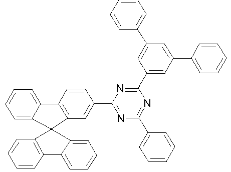 | |
| ETL | |

Zudem weisen die erfindungsgemäßen Verbindungen eine gute Löslichkeit in unpolaren Lösungsmitteln auf und eignen sich folglich für ein Prozessieren aus Lösung. Dadurch werden elektronische Vorrichtungen mit blau fluoreszierenden Emittern erhalten, welche vorteilhafte Leistungsdaten aufweisen.

Alternativ oder zusätzlich können die erfindungsgemäßen Verbindungen auch als Matrixmaterial der Emissionsschicht (EML), als Lochtransportmaterial der Lochtransportschicht (HTL), als Elektronentransportmaterial der Elektronentransportschicht (ETL) oder als Lochinjektionsmaterial einer Lochinjektionsschicht (HIL) einer OLED eingesetzt werden.

## Patentansprüche

1. Verbindung gemäß einer Formel (II-1)oder Formel (II-2) 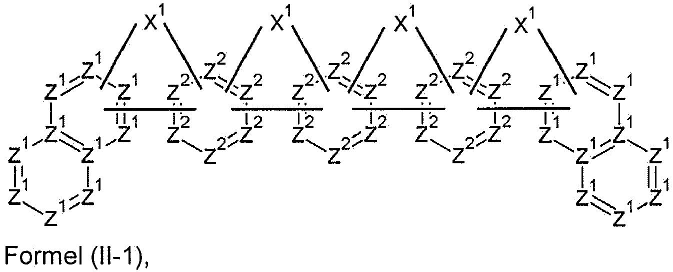 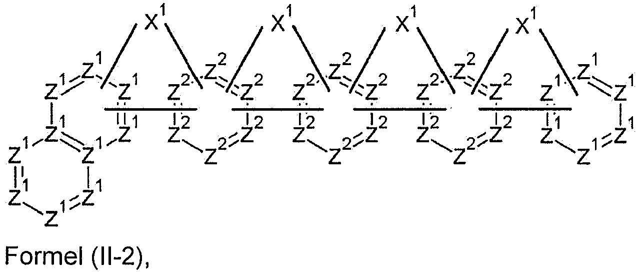 wobei gilt:
Z¹ ist bei jedem Auftreten gleich oder verschieden CR¹ oder N, wobei Z¹ gleich C ist, wenn eine Gruppe gebunden ist;
Z² ist bei jedem Auftreten gleich oder verschieden CR² oder N, wobei Z² gleich C ist, wenn eine Gruppe gebunden ist; und
X¹ ist bei jedem Auftreten gleich oder verschieden BR³, C(R³)₂, C(R³)₂-C(R³)_{2,} -C(R³)₂-O-, -C(R³)₂-S, -R³C=CR³-, -R³C=N-, Si(R³)₂, Ge(R³)₂, -Si(R³)₂-Si(R³)₂-, C=O, O, S, Se, S=O, SO₂, NR³, PR³ oder P(=O)R³, wobei zwei oder mehr Reste R³ miteinander verknüpft sein können und einen Ring bilden können;
R¹ ist bei jedem Auftreten gleich oder verschieden H, D, F, CN, Si(R⁴)₃, eine geradkettige Alkylgruppe mit 1 bis 20 C-Atomen, eine verzweigte oder cyclische Alkylgruppe mit 3 bis 20 C-Atomen, oder ein aromatisches oder heteroaromatisches Ringsystem mit 5 bis 20 aromatischen Ringatomen, wobei die oben genannten Gruppen jeweils mit einem oder mehreren Resten R⁴ substituiert sein können, und wobei eine oder mehrere CH₂-Gruppen in den oben genannten Gruppen durch -C≡C-, -R⁴C=CR⁴, Si(R⁴)₂, C=O, NR⁴, O oder S ersetzt sein kann;
R², R³ ist bei jedem Auftreten gleich oder verschieden H, D, F, Cl, Br, I, C(=O)R⁴, CN, Si(R⁴)₃, N(R⁴)₂, P(=O)(R⁴)₂, OR⁴, S(=O)R⁴, S(=O)₂R⁴, eine geradkettige Alkyl- oder Alkoxygruppe mit 1 bis 20 C-Atomen, eine verzweigte oder cyclische Alkyl- oder Alkoxygruppe mit 3 bis 20 C-Atomen, eine Alkenyl- oder Alkinylgruppe mit 2 bis 20 C-Atomen, ein aromatisches oder heteroaromatisches Ringsystem mit 5 bis 30 aromatischen Ringatomen oder eine Aryloxy- oder Heteroaryloxygruppe mit 5 bis 30 aromatischen Ringatomen, wobei
die oben genannten Gruppen jeweils mit einem oder mehreren Resten R⁴ substituiert sein können, wobei eine oder mehrere CH₂-Gruppen in den oben genannten Gruppen durch -R⁴C=CR⁴-, -C≡C-, Si(R⁴)₂, C=O, C=NR⁴, C(=O)O, C(=O)NR⁴, NR⁴, P(=O)(R⁴), O, S, SO oder SO₂ ersetzt sein können, wobei
ein oder mehrere H-Atome in den oben genannten Gruppen durch D, F, Cl, Br, I oder CN ersetzt sein können;
R⁴ ist bei jedem Auftreten gleich oder verschieden H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, eine geradkettige Alkyl- oder Alkoxygruppe mit 1 bis 20 C-Atomen, eine verzweigte oder cyclische Alkyl- oder Alkoxygruppe mit 3 bis 20 C-Atomen, eine Alkenyl- oder Alkinylgruppe mit 2 bis 20 C-Atomen, ein aromatisches oder heteroaromatisches Ringsystem mit 5 bis 30 aromatischen Ringatomen oder eine Aryloxy- oder Heteroaryloxygruppe mit 5 bis 30 aromatischen Ringatomen, wobei
die oben genannten Gruppen jeweils mit einem oder mehreren Resten R⁵ substituiert sein können, wobei
eine oder mehrere CH₂-Gruppen in den oben genannten Gruppen durch -R⁵C=CR⁵-, -C≡C-, Si(R⁵)₂, C=O, C=NR⁵, C(=O)O, -C(=O)NR⁵-, NR⁵, P(=O)(R⁵), O, S, SO oder SO₂ ersetzt sein können, wobei
ein oder mehrere H-Atome in den oben genannten Gruppen durch D, F, Cl, Br, I oder CN ersetzt sein können, und wobei
zwei oder mehr Reste R⁴ miteinander verknüpft sein können und einen Ring bilden können;
R⁵ ist bei jedem Auftreten gleich oder verschieden H, D, F, Cl, Br, I, CN, eine geradkettige Alkyl- oder Alkoxygruppe mit 1 bis 20 C-Atomen, eine verzweigte oder cyclische Alkyl- oder Alkoxygruppe mit 3 bis 20 C-Atomen, eine Alkenyl- oder Alkinylgruppe mit 2 bis 20 C-Atomen, ein aromatisches oder heteroaromatisches Ringsystem mit 5 bis 30 aromatischen Ringatomen, oder eine Aryloxy- oder Heteroaryloxygruppe mit 5 bis 30 aromatischen Ringatomen, wobei
zwei oder mehr Reste R⁵ miteinander verknüpft sein können und einen Ring bilden können.

2. Verbindung nach Anspruch 1, **dadurch gekennzeichnet, dass** an den Gruppen Ar² die Bindungen zur benachbarten Gruppe Ar¹, bzw. Ar² jeweils in para-Position zueinander vorliegen.

3. Verbindung nach Anspruch 1 oder 2, **dadurch gekennzeichnet, dass** die Gruppen Ar² Phenylgruppen sind, die mit einem oder mehreren Resten R² substituiert sein können.

4. Verbindung nach einem oder mehreren der Ansprüchen 1 bis 3, **dadurch gekennzeichnet, dass** X¹ bei jedem Auftreten gleich oder verschieden gewählt ist aus C(R³)2, -C(R³)₂-C(R³)₂-, -C(R³)₂-O-, Si(R³)₂, O, S und NR³, wobei zwei oder mehr Reste R³ miteinander verknüpft sein können und einen Ring bilden können.

5. Verbindung nach Anspruch 4, **dadurch gekennzeichnet, dass** X¹ gleich C(R³)₂ ist.

6. Verbindung nach einem oder mehreren der Ansprüche 1 bis 5, **dadurch gekennzeichnet, dass** R¹ bei jedem Auftreten gleich oder verschieden gewählt ist aus H, CN und einem aromatischen oder heteroaromatischen Ringsystem mit 5 bis 30 aromatischen Ringatomen, wobei die oben genannten Gruppen jeweils mit einem oder mehreren Resten R⁴ substituiert sein können.

7. Verbindung nach einem oder mehreren der Ansprüchen 1 bis 6, **dadurch gekennzeichnet, dass** R² gleich H oder D ist, wobei R² bevorzugt gleich H ist.

8. Verbindung nach einem oder mehreren der Ansprüche 1 bis 7, **dadurch gekennzeichnet, dass** R³ bei jedem Auftreten gleich oder verschieden gewählt ist aus eine geradkettige Alkylgruppe mit 1 bis 20 C-Atomen oder eine verzweigte Alkylgruppe mit 3 bis 20 C-Atomen und R³ bevorzugt eine geradkettige Alkylgruppe mit 5 bis 12 C-Atomen ist.

9. Verbindung nach einem oder mehreren der Ansprüche 1 bis 8, **dadurch gekennzeichnet, dass** die Verbindung einer der folgenden Formeln (D1) bis (D4) entspricht,

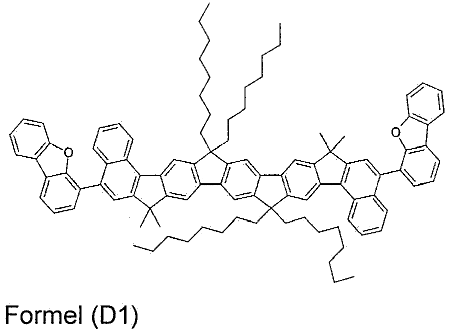

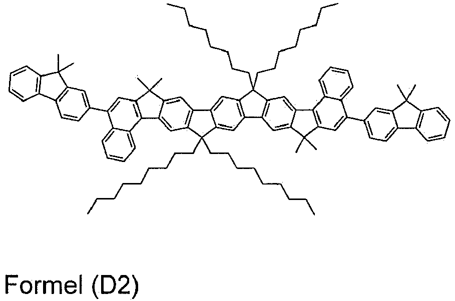

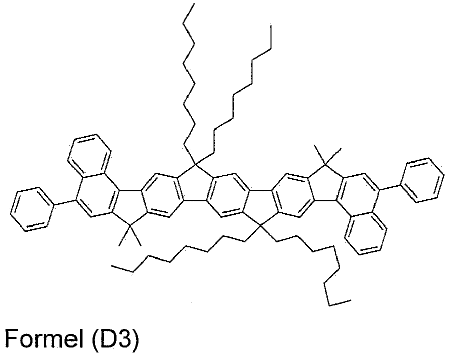

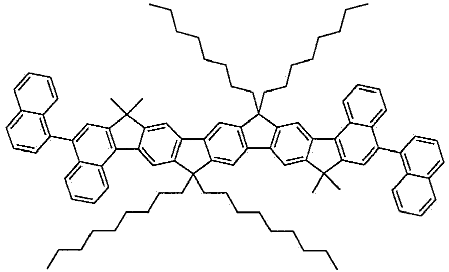

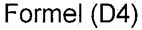

10. Oligomer, Polymer oder Dendrimer enthaltend eine oder mehrere Verbindungen nach einem oder mehreren der Ansprüche 1 bis 9, wobei die Bindung(en) zum Polymer, Oligomer oder Dendrimer an beliebigen, in Formel (I), bzw. Formel (II) mit R¹ oder R² substituierten Positionen lokalisiert sein können.

11. Formulierung, enthaltend mindestens eine Verbindung nach einem oder mehreren der Ansprüche 1 bis 9 oder mindestens ein Polymer, Oligomer oder Dendrimer nach Anspruch 10 sowie mindestens ein Lösungsmittel.

12. Elektronische Vorrichtung, ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), enthaltend mindestens eine Verbindung nach einem oder mehreren der Ansprüche 1 bis 9, oder mindestens ein Oligomer, Polymer oder Dendrimer nach Anspruch 10.

13. Elektronische Vorrichtung nach Anspruch 12, ausgewählt aus organischen Elektrolumineszenzvorrichtungen, enthaltend Kathode, Anode und mindestens eine organische Schicht, wobei die mindestens eine organische Schicht mindestens eine Verbindung nach einem oder mehreren der Ansprüche 1 bis 9 oder mindestens ein Oligomer, Polymer oder Dendrimer nach Anspruch 10 enthält.

14. Elektronische Vorrichtung nach Anspruch 13, **dadurch gekennzeichnet, dass** die Verbindung nach einem oder mehreren der Ansprüche 1 bis 9 oder das Oligomer, Polymer oder Dendrimer nach Anspruch 10 als Lochtransportmaterial in einer Lochtransportschicht, als emittierende Verbindung in einer emittierenden Schicht oder als Matrixverbindung in einer emittierenden Schicht vorliegt, wobei die Verbindung bevorzugt als emittierende Verbindung in einer emittierenden Schicht vorliegt.

15. Verwendung einer Verbindung nach einem oder mehreren der Ansprüche 1 bis 9 oder eines Oligomers, Polymers oder Dendrimers nach Anspruch 10 in einer elektronischen Vorrichtung.

16. Verfahren zur Herstellung einer Verbindung nach einem oder mehreren der Ansprüche 1 bis 9, **dadurch gekennzeichnet, dass** das Verfahren mindestens eine metallkatalysierte Kupplungsreaktion und mindestens eine Ringschlussreaktion umfasst

## Claims

1. Compound of a formula (II-1) or formula (II-2) 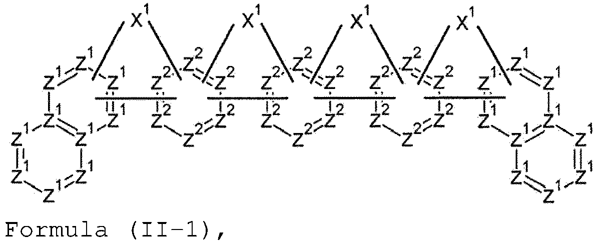 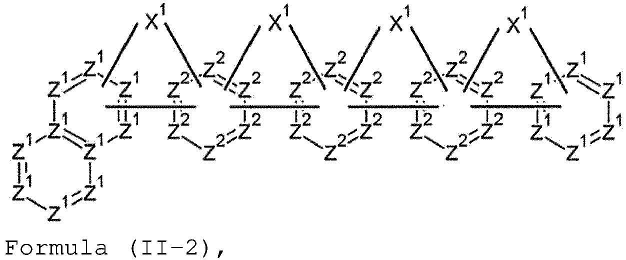 where:
Z¹ is the same or different at each instance and is CR¹ or N, where Z¹ is C when a group is bonded;
Z² is the same or different at each instance and is CR² or N, where Z² is C when a group is bonded; and
X¹ is the same or different at each instance and is BR³, C (R³)₂, C(R³)₂-C(R³)₂, -C(R³)₂-O-, -C(R³)₂-S, -R³C=CR³-, -R³C=N-, Si (R³)₂, Ge(R³)₂, -Si(R³)₂-Si(R³)₂-, C=O, O, S, Se, S=O, SO₂, NR³, PR³ or P(=O)R³, where two or more R³ radicals may be joined to one another and may form a ring;
R¹ is the same or different at each instance and is H, D, F, CN, Si(R⁴)₃, a straight-chain alkyl group having 1 to 20 carbon atoms, a branched or cyclic alkyl group having 3 to 20 carbon atoms, or an aromatic or heteroaromatic ring system having 5 to 20 aromatic ring atoms, where the abovementioned groups may each be substituted by one or more R⁴ radicals, and where one or more CH₂ groups in the abovementioned groups may be replaced by -C=C-, -R⁴C=CR⁴, Si(R⁴)₂, C=O, NR⁴, O or S;
R², R³ is the same or different at each instance and is H, D, F, Cl, Br, I, C(=O)R⁴, CN, Si(R⁴)₃, N(R⁴)₂, P(=O) (R4)₂, OR⁴, S(=O)R⁴, S(=O)₂R⁴, a straight-chain alkyl or alkoxy group having 1 to 20 carbon atoms, a branched or cyclic alkyl or alkoxy group having 3 to 20 carbon atoms, an alkenyl or alkynyl group having 2 to 20 carbon atoms, an aromatic or heteroaromatic ring system having 5 to 30 aromatic ring atoms or an aryloxy or heteroaryloxy group having 5 to 30 aromatic ring atoms, where
the abovementioned groups may each be substituted by one or more R⁴ radicals, where one or more CH₂ groups in the abovementioned groups may be replaced by -R⁴C=CR⁴-, -C=C-, Si(R⁴)₂, C=O, C=NR⁴, C(=O)O, C(=O)NR⁴, NR⁴, P(=O) (R⁴), O, S, SO or SO₂, where
one or more hydrogen atoms in the abovementioned groups may be replaced by D, F, Cl, Br, I or CN;
R⁴ is the same or different at each instance and is H, D, F, Cl, Br, I, C(=O) R⁵, CN, Si(R⁵)₃, N(R⁵)₂, P(=O) (R⁵)₂, OR⁵, S(=O)R⁵, S(=O)₂R⁵, a straight-chain alkyl or alkoxy group having 1 to 20 carbon atoms, a branched or cyclic alkyl or alkoxy group having 3 to 20 carbon atoms, an alkenyl or alkynyl group having 2 to 20 carbon atoms, an aromatic or heteroaromatic ring system having 5 to 30 aromatic ring atoms or an aryloxy or heteroaryloxy group having 5 to 30 aromatic ring atoms, where
the abovementioned groups may each be substituted by one or more R⁵ radicals, where
one or more CH₂ groups in the abovementioned groups may be replaced by -R⁵C=CR⁵-, -C=C-, Si(R⁵)₂, C=O, C=NR⁵, C(=O)O, -C (=O) NR⁵-, NR⁵, P(=O) (R⁵), O, S, SO or SO₂, where
one or more hydrogen atoms in the abovementioned groups may be replaced by D, F, Cl, Br, I or CN, and where two or more R⁴ radicals may be joined to one another and may form a ring;
R⁵ is the same or different at each instance and is H, D, F, Cl, Br, I, CN, a straight-chain alkyl or alkoxy group having 1 to 20 carbon atoms, a branched or cyclic alkyl or alkoxy group having 3 to 20 carbon atoms, an alkenyl or alkynyl group having 2 to 20 carbon atoms, an aromatic or heteroaromatic ring system having 5 to 30 aromatic ring atoms or an aryloxy or heteroaryloxy group having 5 to 30 aromatic ring atoms, where two or more R⁵ radicals may be joined to one another and may form a ring.

2. Compound according to Claim 1, **characterized in that** the bonds to the adjacent Ar¹ or Ar² group in the Ar² groups are each in the para position to one another.

3. Compound according to Claim 1 or 2, **characterized in that** the Ar² groups are phenyl groups which may be substituted by one or more R² radicals.

4. Compound according to one or more of Claims 1 to 3, **characterized in that** X¹ is the same or different at each instance and is selected from C(R³)₂, -C(R³)₂-C(R³)₂-, - C(R³)₂-O-, Si(R³)₂, O, S and NR³, where two or more R³ radicals may be joined to one another and may form a ring.

5. Compound according to Claim 4, **characterized in that** X¹ is C(R³)₂.

6. Compound according to one or more of Claims 1 to 5, **characterized in that** R¹ is the same or different at each instance and is selected from H, CN and an aromatic or heteroaromatic ring system having 5 to 30 aromatic ring atoms, where the abovementioned groups may each be substituted by one or more R⁴ radicals.

7. Compound according to one or more of Claims 1 to 6, **characterized in that** R² is H or D, where R² is preferably H.

8. Compound according to one or more of Claims 1 to 7, **characterized in that** R³ is the same or different at each instance and is selected from a straight-chain alkyl group having 1 to 20 carbon atoms or a branched alkyl group having 3 to 20 carbon atoms and R³ is preferably a straight-chain alkyl group having 5 to 12 carbon atoms.

9. Compound according to one or more of Claims 1 to 8, **characterized in that** the compound corresponds to one of the following formulae (D1) to (D4)

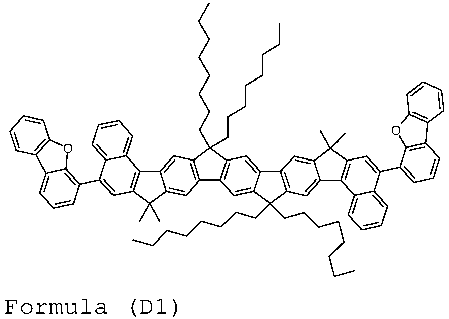

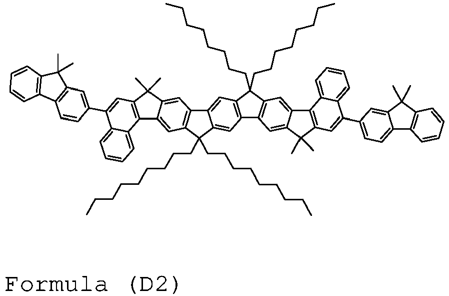

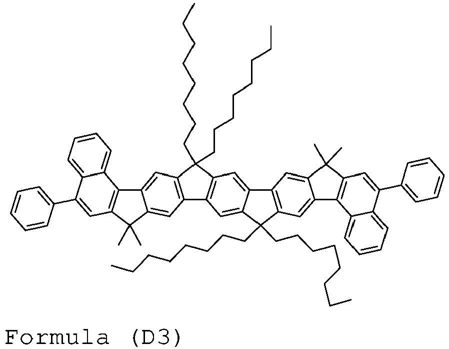

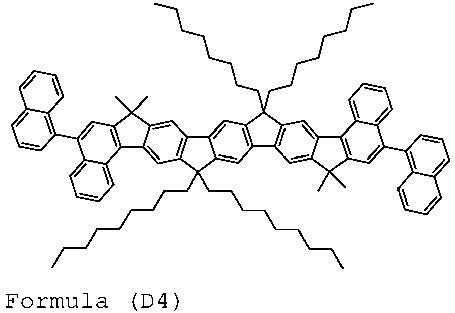

10. Oligomer, polymer or dendrimer containing one or more compounds according to one or more of Claims 1 to 9, wherein the bond(s) to the polymer, oligomer or dendrimer may be localized at any desired positions substituted by R¹ or R² in formula (I) or formula (II).

11. Formulation comprising at least one compound according to one or more of Claims 1 to 9 or at least one polymer, oligomer or dendrimer according to Claim 10 and at least one solvent.

12. Electronic device selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), comprising at least one compound according to one or more of Claims 1 to 9 or at least one oligomer, polymer or dendrimer according to Claim 10.

13. Electronic device according to Claim 12, selected from
organic electroluminescent devices comprising cathode, anode and at least one organic layer, where the at least one organic layer comprises at least one compound according to one or more of Claims 1 to 9 or at least one oligomer, polymer or dendrimer according to Claim 10.

14. Electronic device according to Claim 13, **characterized**
**in that** the compound according to one or more of Claims 1 to 9 or the oligomer, polymer or dendrimer according to Claim 10 is present as hole transport material in a hole transport layer, as emitting compound in an emitting layer or as matrix compound in an emitting layer, where the compound is preferably present as emitting compound in an emitting layer.

15. Use of a compound according to one or more of Claims 1 to 9, or of an oligomer, polymer or dendrimer according to Claim 10, in an electronic device.

16. Process for preparing a compound according to one or more of Claims 1 to 9, **characterized in that** the process comprises at least one metal-catalysed coupling reaction and at least one ring closure reaction.

## Revendications

1. Composé selon une formule (II-1) ou une formule (II-2) 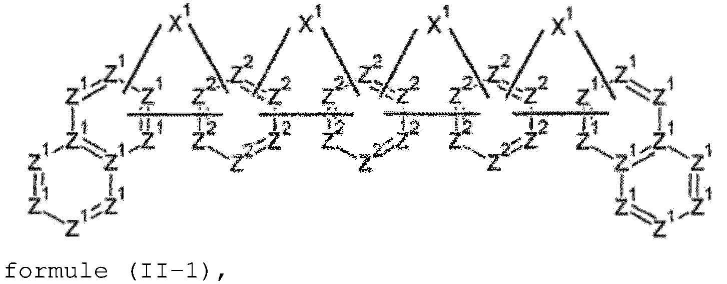 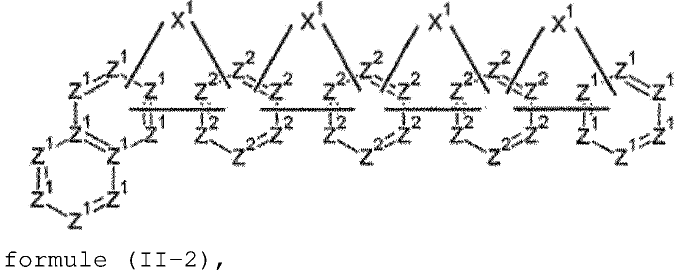 où :
Z¹ est, en chaque occurrence identique ou différent, CR¹ ou N, Z¹ étant égal à C, lorsqu'un groupe y est lié ; et
Z² est, en chaque occurrence identique ou différent, CR² ou N, Z² étant égal à C, lorsqu'un groupe y est lié ; et
X¹ est, en chaque occurrence identique ou différent, BR³, C(R³)₂, C(R³)₂-C(R³)₂, -C(R³)₂-O-, -C(R³)₂-S, -R³C=CR³-, -R³C=N-, Si(R³)₂, Ge(R³)₂, -Si(R³)₂-Si(R³)₂-, C=O, O, S, Se, S=O, SO₂, NR³, PR³ ou P(=O)R³, deux radicaux R³ ou plus pouvant être reliés entre eux et pouvant former un cycle ;
R¹ est, en chaque occurrence identique ou différent, H, D, F, CN, Si(R⁴)₃, un groupe alkyle linéaire comportant 1 à 20 atome(s) de C, un groupe alkyle ramifié ou cyclique comportant 3 à 20 atomes de C, ou un système cyclique aromatique ou hétéroaromatique comportant 5 à 20 atomes de cycle aromatiques, les groupes mentionnés ci-dessus pouvant à chaque fois être substitués par un ou plusieurs radicaux R⁴, et un ou plusieurs groupes CH₂ dans les groupes mentionnés ci-dessus pouvant être remplacés par -C≡C-, -R⁴C=CR⁴-, Si(R⁴)₂, C=O, NR⁴, O ou S ;
R², R³ sont, en chaque occurrence identiques ou différents, H, D, F, Cl, Br, I, C(=O)R⁴, CN, Si(R⁴)₃, N(R⁴)₂, P(=O)(R⁴)₂, OR⁴, S(=O)R⁴, S(=O)₂R⁴, un groupe alkyle ou alcoxyle, linéaire, comportant 1 à 20 atome(s) de C, un groupe alkyle ou alcoxyle, ramifié ou cyclique, comportant 3 à 20 atomes de C, un groupe alcényle ou alcynyle comportant 2 à 20 atomes de C, un système cyclique aromatique ou hétéroaromatique comportant 5 à 30 atomes de cycle aromatiques ou un groupe aryloxyle ou hétéroaryloxyle comportant 5 à 30 atomes de cycle aromatiques,
les groupes mentionnés ci-dessus pouvant à chaque fois être substitués par un ou plusieurs radicaux R⁴, un ou plusieurs groupes CH₂ dans les groupes mentionnés ci-dessus pouvant être remplacés par -R⁴C=CR⁴-, -C≡C-, Si(R⁴)₂, C=O, C=NR⁴, C(=O)O, C(=O)NR⁴, NR⁴, P(=O)(R⁴), O, S, SO ou SO₂,
un ou plusieurs atomes H dans les groupes mentionnés ci-dessus pouvant être remplacés par D, F, Cl, Br, I ou CN ;
R⁴ est, en chaque occurrence identique ou différent, H, D, F, Cl, Br, I, C(=O)R⁵, CN, Si(R⁵)₃, N(R⁵)₂, P(=O) (R⁵)₂, OR⁵, S(=O)R⁵, S(=O)₂R⁵, un groupe alkyle ou alcoxyle, linéaire, comportant 1 à 20 atome(s) de C, un groupe alkyle ou alcoxyle, ramifié ou cyclique, comportant 3 à 20 atomes de C, un groupe alcényle ou alcynyle comportant 2 à 20 atomes de C, un système cyclique aromatique ou hétéroaromatique comportant 5 à 30 atomes de cycle aromatiques ou un groupe aryloxyle ou hétéroaryloxyle comportant 5 à 30 atomes de cycle aromatiques,
les groupes mentionnés ci-dessus pouvant à chaque fois être substitués par un ou plusieurs radicaux R⁵,
un ou plusieurs groupes CH₂ dans les groupes mentionnés ci-dessus pouvant être remplacés par -R⁵C=CR⁵-, -C≡C-, Si(R⁵)₂, C=O, C=NR⁵, C(=O)O, -C(=O)NR⁵-, NR⁵, P(=O)(R⁵), O, S, SO ou SO₂,
un ou plusieurs atomes H dans les groupes mentionnés ci-dessus pouvant être remplacés par D, F, Cl, Br, I ou CN, et
deux radicaux R⁴ ou plus pouvant être reliés entre eux et pouvant former un cycle ;
R⁵ est, en chaque occurrence identique ou différent, H, D, F, Cl, Br, I, CN, un groupe alkyle ou alcoxyle, linéaire, comportant 1 à 20 atome (s) de C, un groupe alkyle ou alcoxyle, ramifié ou cyclique, comportant 3 à 20 atomes de C, un groupe alcényle ou alcynyle comportant 2 à 20 atomes de C, un système cyclique aromatique ou hétéroaromatique comportant 5 à 30 atomes de cycle aromatiques ou un groupe aryloxyle ou hétéroaryloxyle comportant 5 à 30 atomes de cycle aromatiques,
deux radicaux R⁵ ou plus pouvant être reliés entre eux et pouvant former un cycle.

2. Composé selon la revendication 1, **caractérisé en ce qu'**au niveau des groupes Ar², les liaisons au groupe voisin Ar¹ ou, selon le cas, Ar² se trouvent à chaque fois en position para l'une par rapport à l'autre.

3. Composé selon la revendication 1 ou 2, **caractérisé en ce que** les groupes Ar² sont des groupes phényle, qui peuvent être substitués par un ou plusieurs radicaux R².

4. Composé selon l'une ou plusieurs des revendications 1 à 3, **caractérisé en ce que** X¹ est choisi en chaque occurrence de manière identique ou différente parmi C(R³)₂, -C(R³)₂-C(R³)₂-, -C(R³)₂-O-, Si(R³)₂, O, S, et NR³, deux radicaux R³ ou plus pouvant être reliés entre eux et pouvant former un cycle.

5. Composé selon la revendication 4, **caractérisé en ce que** X¹ est égal à C(R³)₂.

6. Composé selon l'une ou plusieurs des revendications 1 à 5, **caractérisé en ce que** R¹ est choisi en chaque occurrence de manière identique ou différente parmi H, CN et un système cyclique aromatique ou hétéroaromatique comportant 5 à 30 atomes de cycle aromatiques, les groupes mentionnés ci-dessus pouvant à chaque fois être substitués par un ou plusieurs radicaux R⁴.

7. Composé selon l'une ou plusieurs des revendications 1 à 6, **caractérisé en ce que** R² est égal à H ou D, R² étant préférablement égal à H.

8. Composé selon l'une ou plusieurs des revendications 1 à 7, **caractérisé en ce que** R³ est choisi en chaque occurrence de manière identique ou différente parmi un groupe alkyle linéaire comportant 1 à 20 atome(s) de C ou un groupe alkyle ramifié comportant 3 à 20 atomes de C et R³ est préférablement un groupe alkyle linéaire comportant 5 à 12 atomes de C.

9. Composé selon l'une ou plusieurs des revendications 1 à 8, **caractérisé en ce que** le composé correspond à une des formules suivantes (D1) à (D4),

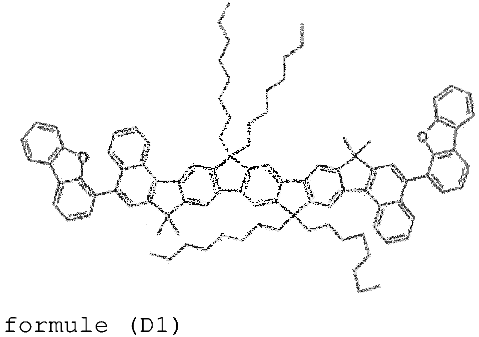

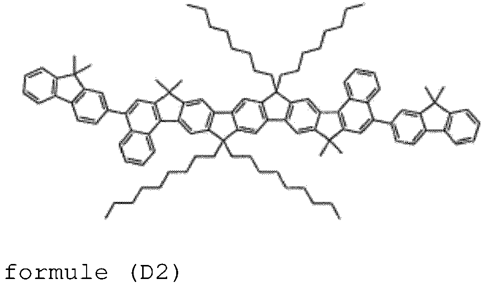

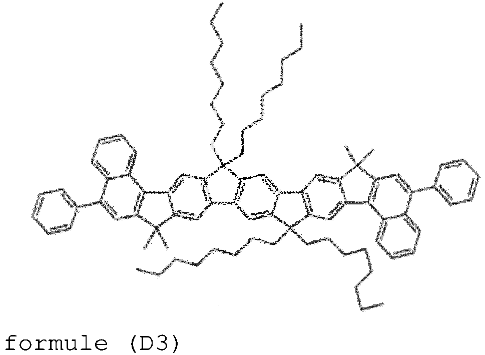

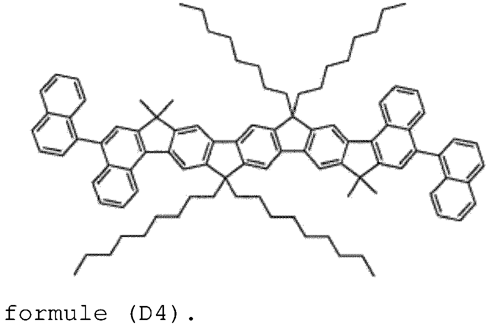

10. Oligomère, polymère ou dendrimère contenant un ou plusieurs composés selon l'une ou plusieurs des revendications 1 à 9, la ou les liaison (s) au polymère, à l'oligomère ou au dendrimère pouvant être localisées au niveau de quelconques positions substituées par R¹ ou R² dans la formule (I), respectivement dans la formule (II).

11. Formulation, contenant au moins un composé selon l'une ou plusieurs des revendications 1 à 9, ou au moins un polymère, oligomère ou dendrimère selon la revendication 10 ainsi qu'au moins un solvant.

12. Dispositif électronique, choisi dans le groupe constitué par les circuits intégrés organiques (OIC), les transistors organiques à effet de champ (OFET), les transistors organiques à film mince (OTFT), les transistors électroluminescents organiques (OLET), les cellules solaires organiques (OSC), les détecteurs optiques organiques, les photorécepteurs organiques, les dispositifs organiques d'extinction de champ (OFQD), les cellules électrochimiques électroluminescentes organiques (OLEC), les diodes laser organiques (O-laser) et les dispositifs électroluminescents organiques (OLED), contenant au moins un composé selon l'une ou plusieurs des revendications 1 à 9, ou au moins un oligomère, polymère ou dendrimère selon la revendication 10.

13. Dispositif électronique selon la revendication 12, choisi par des dispositifs électroluminescents organiques, contenant cathode, anode et au moins une couche organique, l'au moins une couche organique contenant au moins un composé selon l'une ou plusieurs des revendications 1 à 9, ou au moins un oligomère, polymère ou dendrimère selon la revendication 10.

14. Dispositif électronique selon la revendication 13, **caractérisé en ce que** le composé selon l'une ou plusieurs des revendications 1 à 9 ou l'oligomère, le polymère ou le dendrimère selon la revendication 10 est présent en tant que matériau de transport de trou dans une couche de transport de trou, en tant que composé émetteur dans une couche émettrice ou en tant que composé de matrice dans une couche émettrice, le composé étant présent préférablement en tant que composé émetteur dans une couche émettrice.

15. Utilisation d'un composé selon l'une ou plusieurs des revendications 1 à 9 ou d'un oligomère, d'un polymère ou d'un dendrimère selon la revendication 10 dans un dispositif électronique.

16. Procédé pour la préparation d'un composé selon l'une ou plusieurs des revendications 1 à 9, **caractérisé en ce que** le procédé comprend au moins une réaction de couplage catalysée par un métal et au moins une réaction de cyclisation.
